# EUROPEAN PATENT SPECIFICATION

(11) **EP 3 277 818 B1**
(45) Date of publication and mention of the grant of the patent: **08.01.2020**
(21) Application number: 16719778.9
(22) Date of filing: 01.04.2016
(51) Int. Cl.: C12N 15/63, C07K 14/705, C12N 15/85, G01N 33/68, C12N 15/67

(54) **USE OF VITAMINS AND VITAMIN METABOLIC GENES AND PROTEINS FOR RECOMBINANT PROTEIN PRODUCTION IN MAMMALIAN CELLS**
VERWENDUNG VON VITAMINEN UND VITAMINSTOFFWECHSELGENEN UND PROTEINEN ZUR PRODUKTION VON REKOMBINANTEN PROTEINEN IN SÄUGETIERZELLEN
UTILISATION DE VITAMINES ET DE GÈNES MÉTABOLIQUES DE VITAMINE AINSI QUE DE PROTÉINES POUR LA PRODUCTION DE PROTÉINES DE RECOMBINAISON DANS DES CELLULES DE MAMMIFÈRES

(30) Priority: 03.04.2015 US 201562142516 P
(43) Date of publication of application: 07.02.2018
(73) Proprietor: Selexis S.A., 1228 Plan-les-Ouates (CH)
(72) Inventor: MERMOD, Nicolas, 1228 Plan-les-Ouates (CH); POURCEL, Lucille, 1228 Plan-les-Ouates (CH); GIROD, Pierre-Alain, 1228 Plan-les-Ouates (CH); LE FOURN, Valérie, 1228 Plan-les-Ouates (CH)
(74) Representative: IPrime Rentsch Kaelin AG
(86) International application number: PCT/EP2016/057228
(87) International publication number: WO 2016/156574

(56) References cited:
- WO-A1-2009/080759
- WO-A1-2010/097240
- CACCIATORE J J ET AL: "Gene amplification and vector engineering to achieve rapid and high-level therapeutic protein production using the Dhfr-based CHO cell selection system", BIOTECHNOLOGY ADVANCES, ELSEVIER PUBLISHING, BARKING, GB, vol. 28, no. 6, 1 November 2010 (2010-11-01), pages 673-681, XP027331807, ISSN: 0734-9750 [retrieved on 2010-04-21]
- ROTHEM L ET AL: "THE REDUCED FOLATE CARRIER GENE IS A NOVEL SELECTABLE MARKER FOR RECOMBINANT PROTEIN OVEREXPRESSION", MOLECULAR PHARMACOLOGY, AMERICAN SOCIETY FOR PHARMACOLOGY AND EXPERIMENTAL THERAPEUTICS, US, vol. 68, no. 3, 1 September 2005 (2005-09-01), pages 616-624, XP009063198, ISSN: 0026-895X
- JIANWEI ZHU: "Mammalian cell protein expression for biopharmaceutical production", BIOTECHNOLOGY ADVANCES., vol. 30, no. 5, 1 September 2012 (2012-09-01), pages 1158-1170, XP055276897, GB ISSN: 0734-9750, DOI: 10.1016/j.biotechadv.2011.08.022
- TINGFENG LAI ET AL: "Advances in Mammalian Cell Line Development Technologies for Recombinant Protein Production", PHARMACEUTICALS, vol. 6, no. 5, 26 April 2013 (2013-04-26), pages 579-603, XP055290466, DOI: 10.3390/ph6050579
- Jason Dean ET AL: "Metabolic Analysis of Antibody Producing CHO Cells in Fed-Batch Production", Biotechnol. Bioeng, 1 January 2013 (2013-01-01), pages 1735-1747, XP055206007, Retrieved from the Internet: URL:http://onlinelibrary.wiley.com/store/1 0.1002/bit.24826/asset/24826_ftp.pdf?v=1&t =icvxj4ou&s=7acb646394bc863bbe01e739073891 259d82e726 [retrieved on 2015-08-03] cited in the application

## Description

### BACKGROUND OF THE INVENTION

Vitamins are essential micronutrients required to support cell growth and propagation. Mammalian cells can not synthesize them and mammals must therefore obtain them from their diet. In contrast, bacteria, fungi, and plants synthesize vitamins. The main function of vitamins is to act as cofactors or coenzymes in various enzymatic reactions such as the TCAcycle, glycolysis, amino acid synthesis and Acetyl-CoA biosynthesis.

Vitamin deficiency is directly linked to numerous diseases. For example, acute deficiency of vitamin B1 in humans leads to a disease called beriberi, which in turn can result in fatal neurological and cardiovascular disorders. Moreover, mice lacking genes involved in vitamin uptake display severe symptoms. For instance, the knockout of the vitamin B1 mitochondrial transporter S1c25a19 causes embryo lethality, CNS malformations and anemia (Lindhurst et al., 2006). Mice lacking the vitamin H and B5 (pantothenate) transporter exhibit growth retardation, decreased bone density, decreased bone length, and lethality after 10 weeks (Ghosal et al., 2012). Deficiency of cytoplasmic or mitochondrial activities that may be linked to vitamin metabolism may also alter cell or organism functions. For instance, the knock-out of murine pantothenate kinase genes (PANK) leads to defect in mitochondria and cellular respiration as well as coenzyme A deficiency (Brunetti *et al.* 2012; Garcia *et al.,* 2012).

Chinese hamster ovary (CHO) cells are widely used in industrial processes for the production of recombinant therapeutic proteins. The viability of CHO cells and other eukaryotic cells used in industrial processes (NS0, baby hamster kidney (BHK) and human embryo kidney-293 (HEK-293)) are dependent on vitamin uptake. Similarly, primary cells such as human cells for gene or cell-based therapies and for regenerative medicine, are also dependent on vitamin uptake.

Optimization of cell culture media and cell lines is often performed in order to obtain a higher yield of recombinant proteins. Recent studies determining changes in central metabolism that accompany growth and monoclonal antibody production highlighted a regulatory link between cell metabolism, media metabolites and cell growth (Dean et al., 2013). For instance, work has focused on controlling the cell division cycle by depleting specific nutrients or by directly controlling cell cycle regulators, as excessive cell growth and division negatively affects the protein production yields (see Du et al., 2014, and references therein). However, these interventions are often accompanied by unwanted effects on the quality and/or on the post-translational processing of the recombinant protein (Nam et al., 2008; Sajan et al., 2010; Sampathkumar et al., 2006; Trummer et al., 2006).

Other efforts to improve selection of transformed cell lines concentrated on the development of new molecular markers that do not require any resistance to toxic antibiotic compound. For instance, the increased expression of components of the nucleotide or amino acid biosynthetic pathways, such as dehydrofolate reductase or glutamine synthase, have been used for the metabolic selection of recombinant protein-expressing cells, by inclusion of their coding sequences in expression vectors (Cacciatore et al. 2010, Birch and Racher 2006, WO2009/080759; US Patent Publication 20100330572. For instance, the coding sequence of a folate transporter was used to select for increased transgene expression (Rothem et al., 2005). Although this approach has yielded increased expression of proteins of pharmacological interest, several studies reported unstable expression levels, for instance when used to amplify the transgene copy number (Schlatter et al., 2005; Chusainow et al., 2009).

WO 2010/097240 A1 relates to a selection system for selecting host cells that express a product of interest. It is aimed at providing a selection system with a high stringency for selecting host cells producing a product of interest with high yield.

Mammalian cell metabolism and growth may also directly depend on vitamin availability. Thus, there is a need in the art to modulate the metabolism and/or growth of cultured cells by controlling the vitamin uptake, expression of vitamin metabolic genes and/or the concentration of specific vitamins in the culture media, generally with the aim of improved therapeutic protein expression. There is also a need for alternative cell selection methods. The present invention is directed at addressing one or more of these needs as well as other needs in the art.

### BRIEF DESCRIPTION OF THE FIGURES

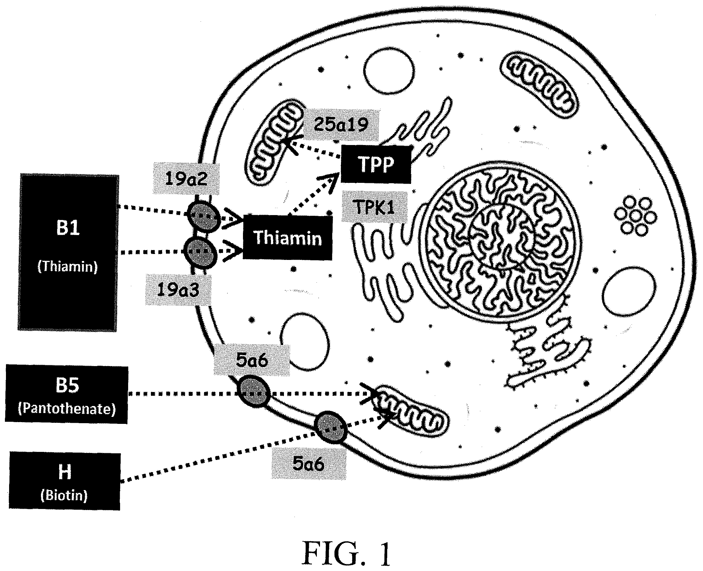

**Fig. 1** provides an overview of the vitamin transport into mammalian cells and organelles.

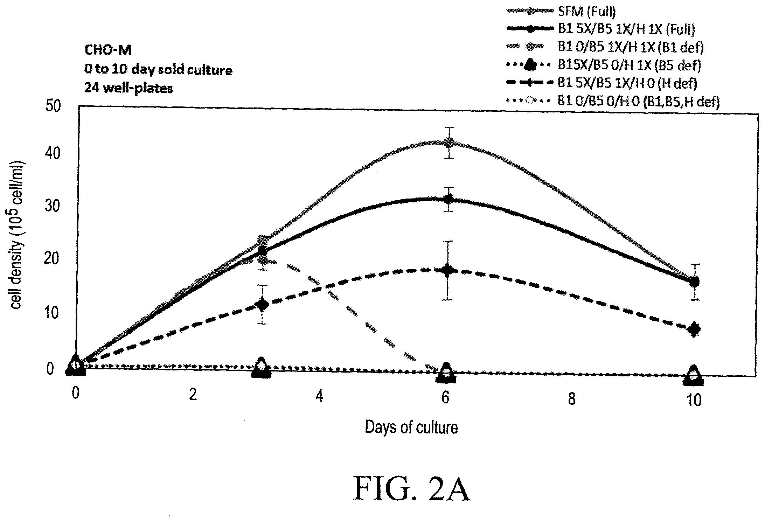

**Fig. 2A-B** show CHO-M growth in vitamin-depleted relative to non-depleted media. Cells were seeded to 50000 cells/ml into 500 µl of B-CDmin culture medium supplemented or not supplemented with vitamin B1, B5 or H (see Table 2), or in a complete medium (SFM). The cells were cultivated in 24 well-plates for the indicated time without shaking. Cell density (A) and viability (trypan blue exclusion assay, panel (B)) was measured after at 3, 6 and 10 days of culture.

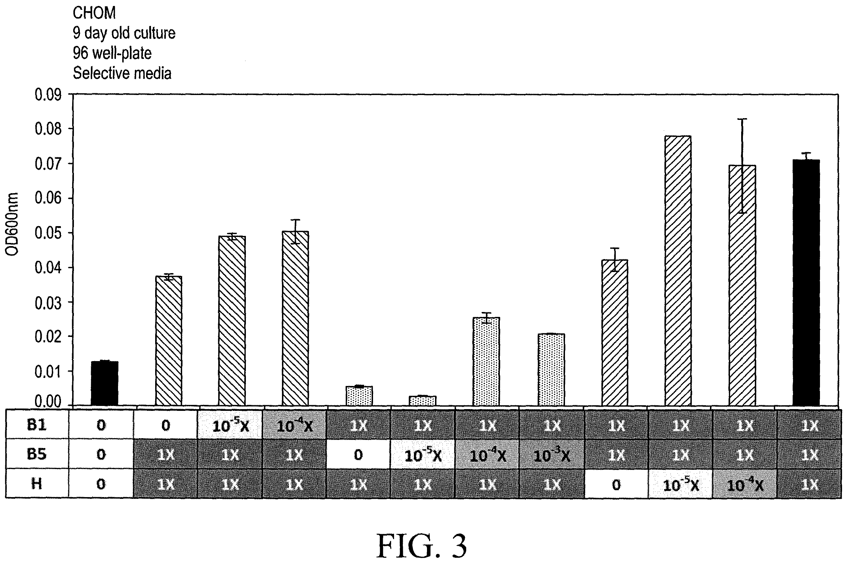

**Fig. 3** shows CHO-M growth in vitamin-depleted media. Cells (5000 cells/ml) were transferred into 150 µl media with different concentrations of vitamin B1, B5 or H (from 0 to 1X, see Table 2). 96 well-plates were used and growth was measured after 9 days of culture, by measuring the OD at 600nm. Hatched (forward-leaning), dotted dark and hatched (backward-leaning) bars indicate modulation of B1, B5 and H concentrations, respectively.

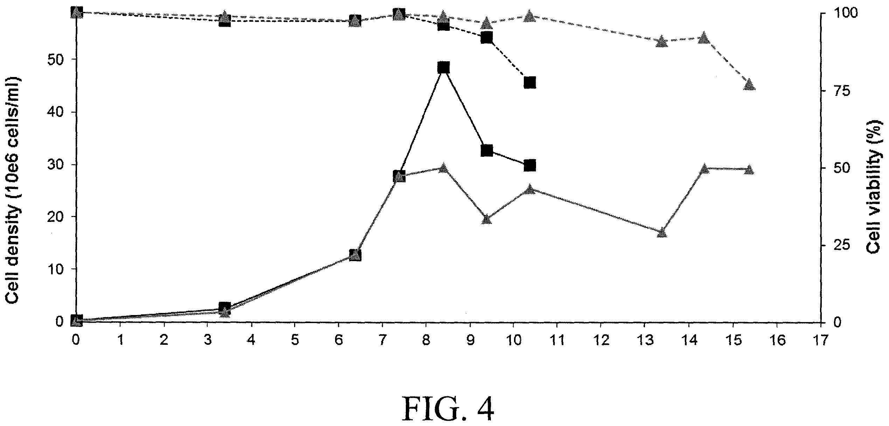

**Fig. 4** shows the effect of vitamin B5 depletion on cell growth and viability of an antibody-secreting CHO-M cell clone fed-batch culture. A trastuzumab-secreting CHO-M cell clone was grown in complete medium (black squares), or in a vitamin depleted medium (grey triangles, 5000:1 V:V mix of B-CDmin and full CD medium), both supplemented with 6mM glutamine. Feeds of the same culture medium were added at day 3, 6, 7, 8, 9, 10 and 13. Cultures were analyzed for the viable cell density (VCD, continuous lines) and for cell viability (% viable cells, dotted lines).

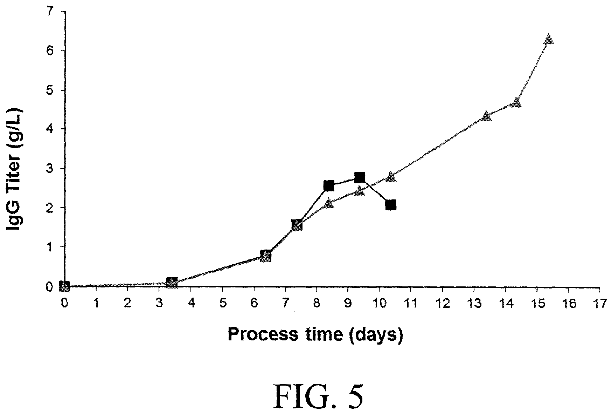

**Fig. 5** shows the effect of vitamin B5 depletion on the immunoglobulin titer of a CHO-M cell clone in fed-batch cultures. The trastuzumab-secreting CHO-M cell clone grown in complete (black squares) or in the vitamin B5 depleted medium (grey triangles) of Fig. 4 was assayed for the titer of antibody secreted in the culture medium by a double sandwich ELISA assay.

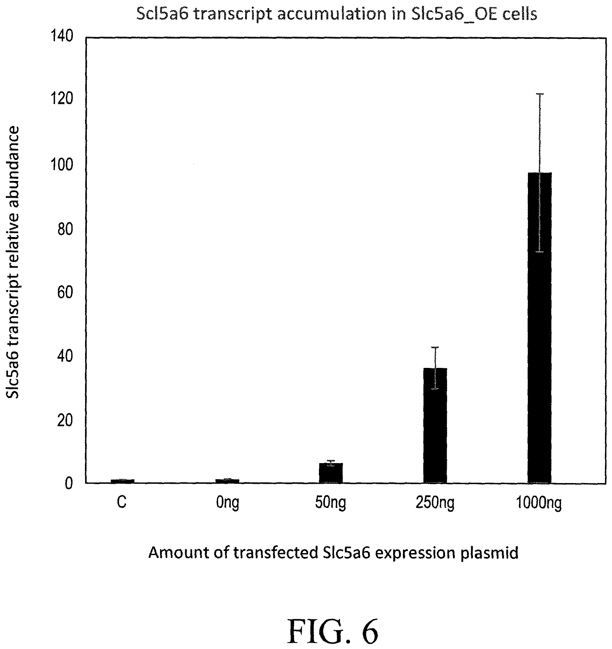

**Fig. 6** shows *SLC5A6* mRNA levels in CHO-M stable lines. Polyclonal populations transfected with the indicated amount of the Slc5a6 expression vector were selected for puromycin resistance, and the mRNA levels of Slc5a6 were determined by RT-qPCR. *SLC5A6* transcript accumulation was normalized to that of the *GAPDH* mRNA, and it is represented relative to the endogenous *SLC5A6* mRNA level of untransformed cells used as control which was set to 1. 0ng indicates cells transfected solely by GFP and puromycin resistance expression vectors, whereas C stands for untransformed control cells.

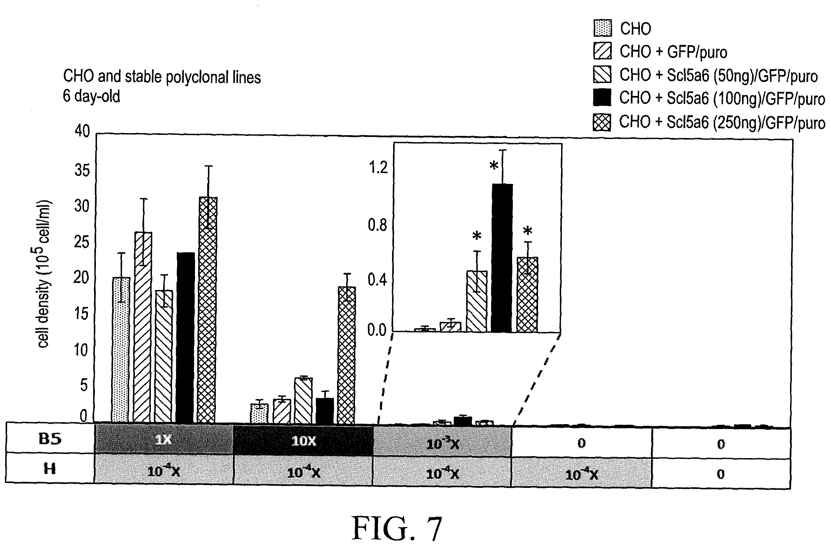

**Fig. 7** shows the effect of *SLC5A6* on CHO-M growth in vitamin-limiting conditions. Cells were seeded at 20000 cells/ml into 500µl of B-CD min media supplemented with the indicated amounts of vitamin B5 and H, and with B1 (1X). A 24 well-plate was used and growth was measured after 6 days of culture by measuring viable cell density. Stars represent a significant difference (p<0.05) between the transfected and non-transfected cells within the same condition of growth and culture media.

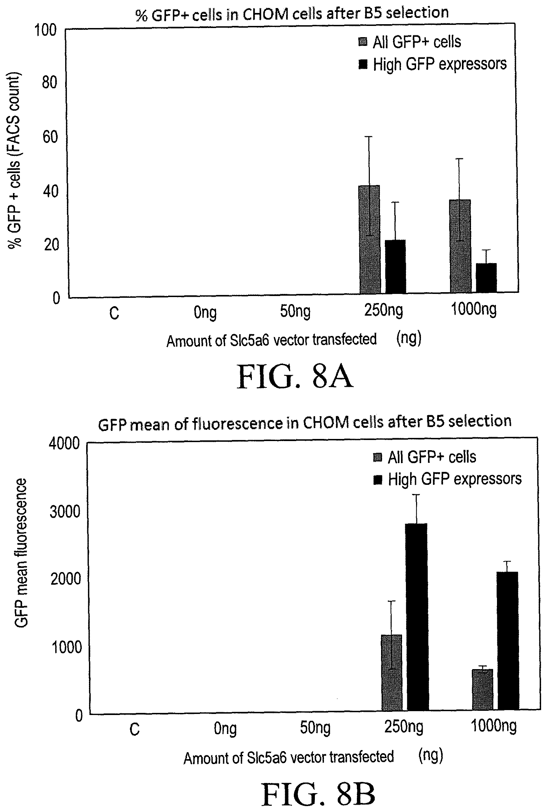

**Fig. 8A-B** show the selection of CHO-M transfected cells using B5 deficient media and SLC5A6 transporter. Increasing amounts of Slc5a6 vector were transfected (0, 50, 250 and 1000ng), together with GFP and puromycin plasmids. After selection of the stable polyclonal population in B5 deficient medium (10⁻³X), all GFP+ expressing cells (grey bars: all GFP+ cells) and high GFP expressors (black bars, high GFP expressors) were quantified by FACS **(A).** Mean of fluorescence for the same cells was also quantified by FACS **(B).** C indicates untransformed CHO-M cells used as control. The number of cells surviving the selection process was too low for quantification upon the transfection of carrier DNA (0ng) or 50 ng of the Slc5a6 expression vector.

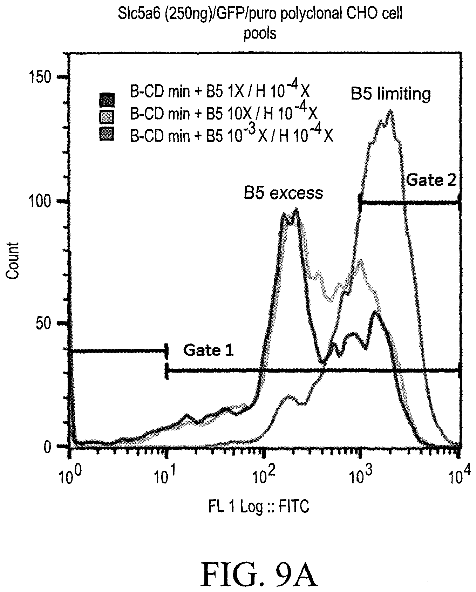

**Fig. 9A-C** show FACS graphs representing enrichment of all GFP+ and of high GFP-expressing cells from stable polyclonal cell populations co-transfected with the SLC5A6, GFP and puromycin resistance (puro) expression plasmids. Transfected cells were submitted to a first selection with puromycin, followed by a second selection by culture in media containing either an excess (B5 10X / H 10⁻⁴X) or limiting (B5 10⁻³X / H 10⁻⁴X) vitamin B5 concentration, or they were cultivated with the non-selective culture medium (B5 1X / H 10⁻⁴X) as a control. (A): FACS profiles of the GFP fluorescence of transfected CHO-M after cultivating the cells for 7 days in the media containing different concentration of B5 (10⁻³ X, 1X or 10X), as indicated. Gate 1 represents all GFP expressing cells, while Gate 2 is restricted to the highest GFP-expressing cells. Enrichment of GFP+ fluorescent cells **(B)** and the geometric mean of the GFP fluorescence of the cells (C) are represented for polyclonal cell pools co-transfected with various amounts of Slc5a6 expression vectors (e.g. 0ng, 100ng and 250ng, as indicated), and with the GFP and the puromycin resistance vectors.

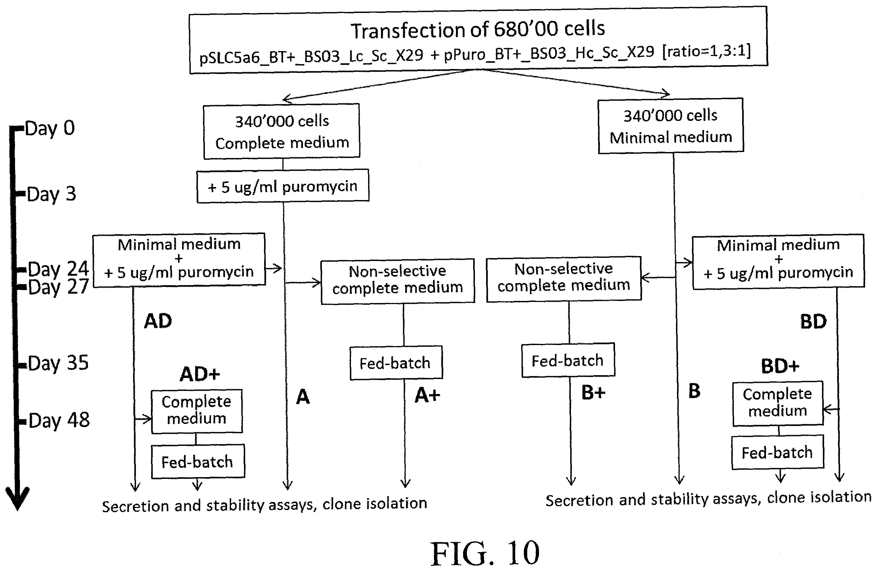

**Fig. 10** shows an experimental cell selection workflow. CHO-M cells were co-transfected with the *SLC5A6* and IgG light chain plasmid and with a puromycin resistance and IgG heavy chain construct, after which the culture was split and selected either in presence of puromycin (**condition A**) or in the vitamin deprived culture medium (minimal medium, **condition B**), or by a double selection (**AD and BD**). This was followed by immunoglobulin secretion assays of the resulting polyclonal cell pools. After selection, part of the cells were transferred to a non-selective culture medium, for passage during a 10-weeks study of the stability of expression (A+, B+), or in fed batch bioreactors (AD+ and BD+).

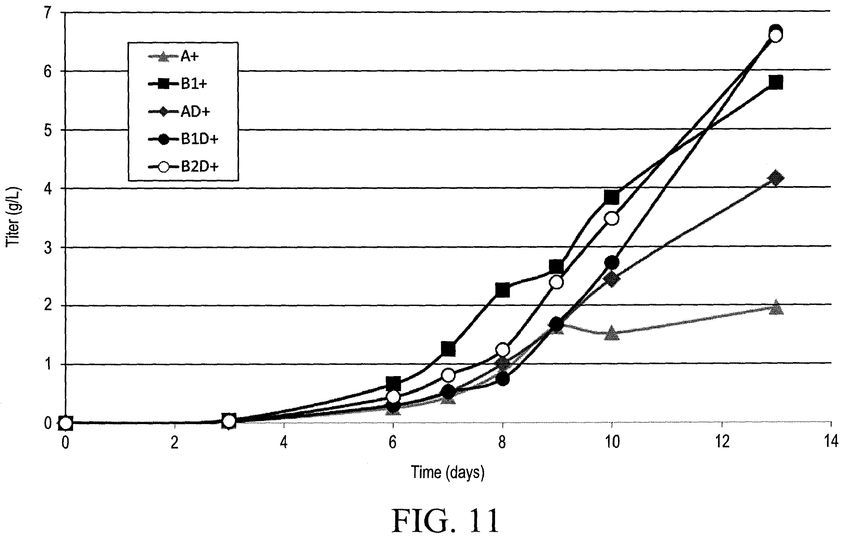

**Fig. 11** shows immunoglobulin secretion of cell populations selected using puromycin or vitamin depletion, or using both selections. Total polyclonal cell pools were selected by growth in the complete medium containing 5 µg/ml puromycin (A+), in the minimal medium (B1+), or by the double selection, in the minimal medium and in presence of 5 µg/ml of puromycin (B1D+ and B2D+), as depicted in Fig. 10. Two independent cell populations were analyzed for the B selection regimen, termed B1 and B2, yielding the doubly selected B1D+ and B2D+ populations, respectively. Two independent populations were analyzed for the BD+ selection regimen. Selected pools were grown in complete medium, and feeds were added at day 3, and at days 6 to 10. Samples were analyzed for the titer of secreted antibody by double sandwich ELISA.

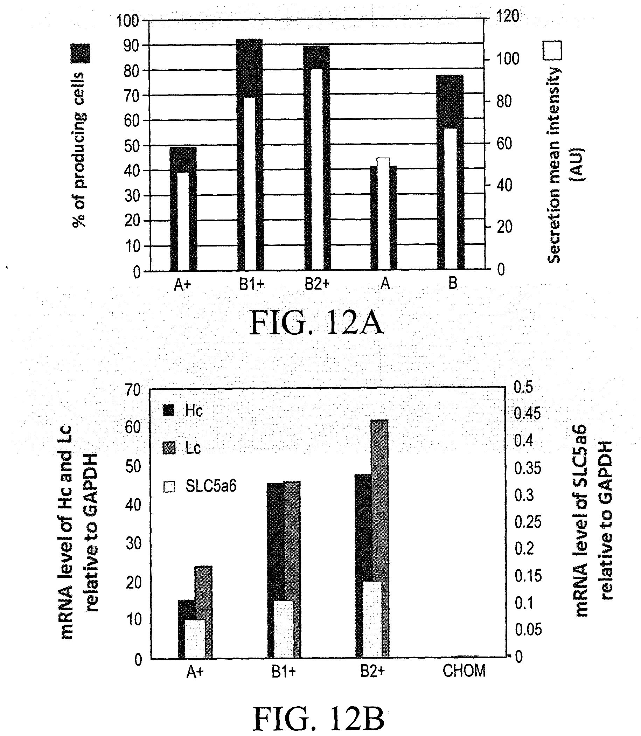

**Fig. 12** shows the immunoglobulin secretion of cell populations selected using puromycin or vitamin depletion. **(A)** Cells were analyzed at day 39 of culture following the selection regimen depicted in Fig. 10. Cell secretion was detected by a fluorescent antibody complex that binds the secreted therapeutic antibody that is displayed at the cell surface and secreted. The black bars and left-hand scale indicate the percentage of cells that secrete the antibody in the polyclonal populations. The cell surface fluorescence mean intensity, indicative of the secretion level of individual cells, is displayed as white bars in arbitrary units (AU) on the right hand-side scale. Two independent populations were analyzed for the B+ selection regimen (B1+, B2+). **(B)** The cell populations of panel A were analyzed for the mRNA levels of the heavy chain (Hc) and light chain (Lc) of the IgG, or of the vitamin B5 transporter (SLC5A6).

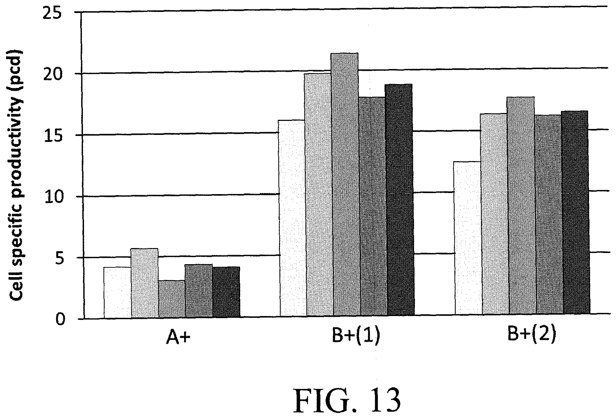

**Fig. 13** shows the immunoglobulin secretion stability of populations selected using puromycin or vitamin depletion. The polyclonal cell pools from Fig. 8, selected using puromycin (A+) or by vitamin B5 deprivation (B+), were maintained in complete medium and passaged twice a week for expression stability studies. The specific productivity of the cell populations, expressed in picogram of secreted antibody per cell and per day (pcd), was measured after each passage for 10 weeks.

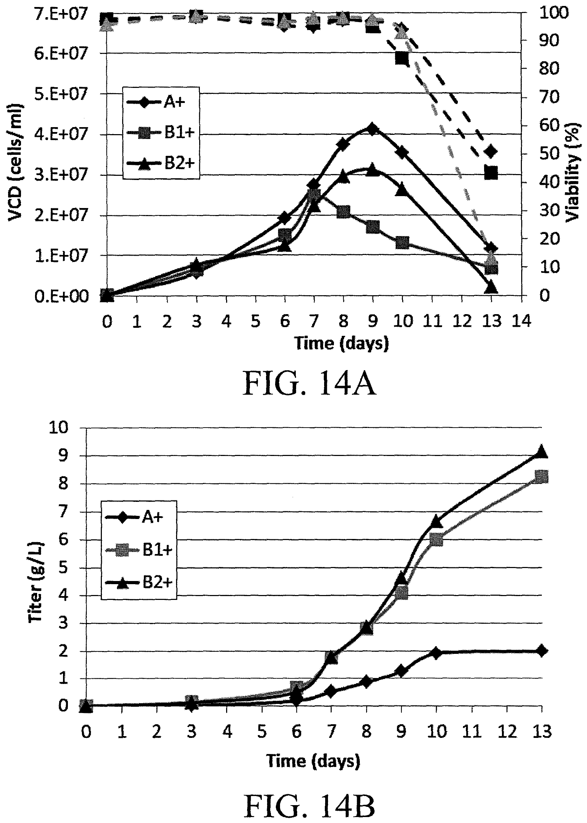

**Fig. 14** shows the immunoglobulin production assays of fed-batch cultures of cell populations selected using puromycin or vitamin depletion. Selected pools were grown in complete medium in fed-batch cultures, and feeds were added at day 3, and at days 6 to 10. Samples were analyzed for viable cell density (VCD) and for viability (% viable cells, dotted lines) (A), and for the titer of secreted antibody by double sandwich ELISA **(B).**

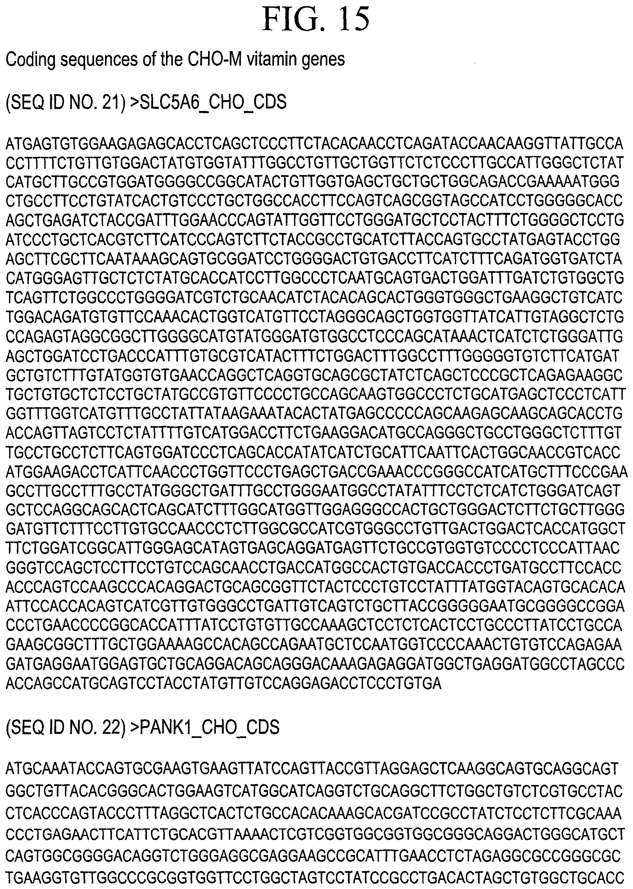

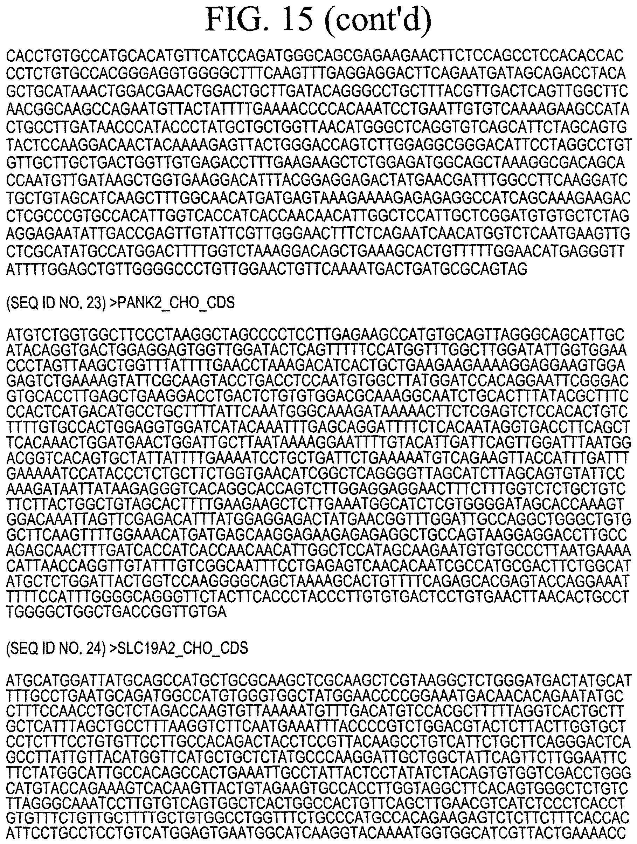

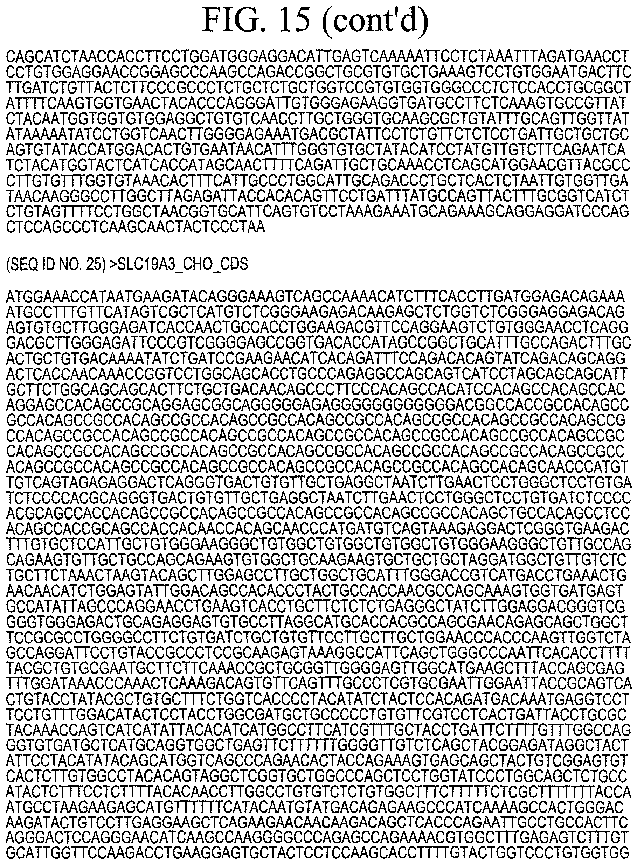

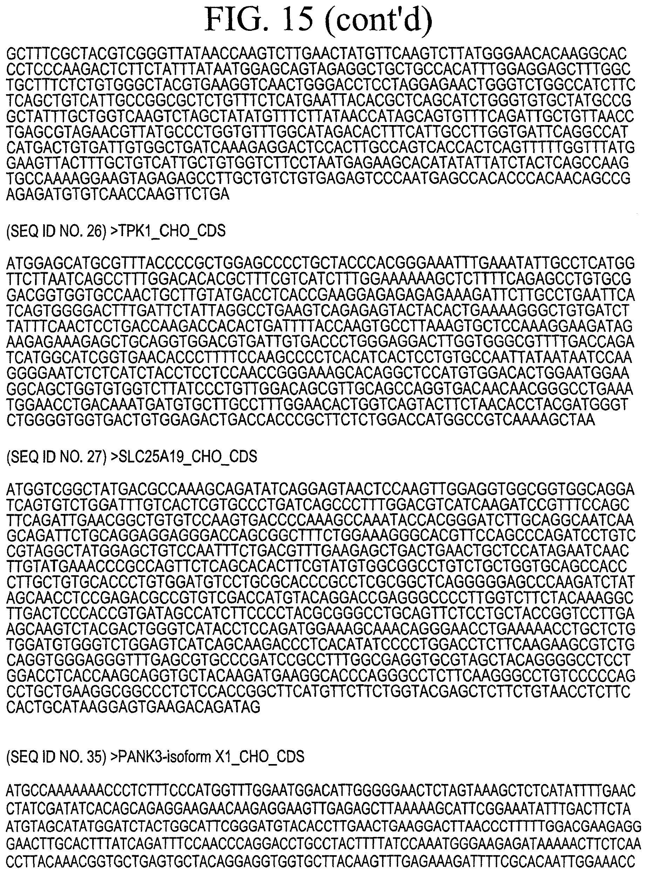

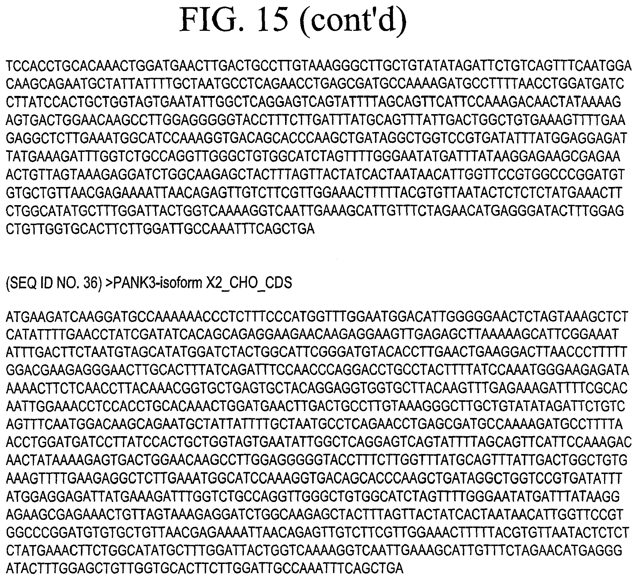

**Fig. 15** shows the coding sequences (CDSs) of different CHO-M vitamin genes.

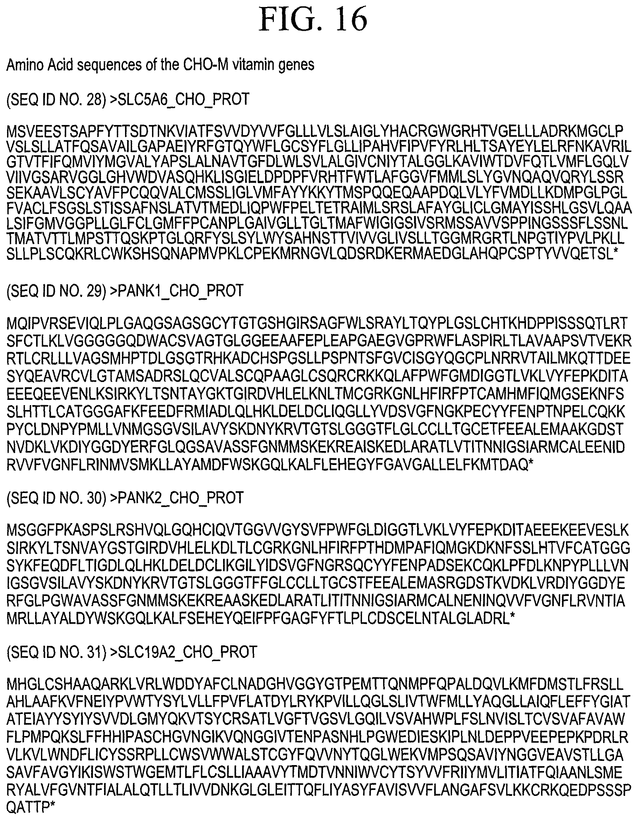

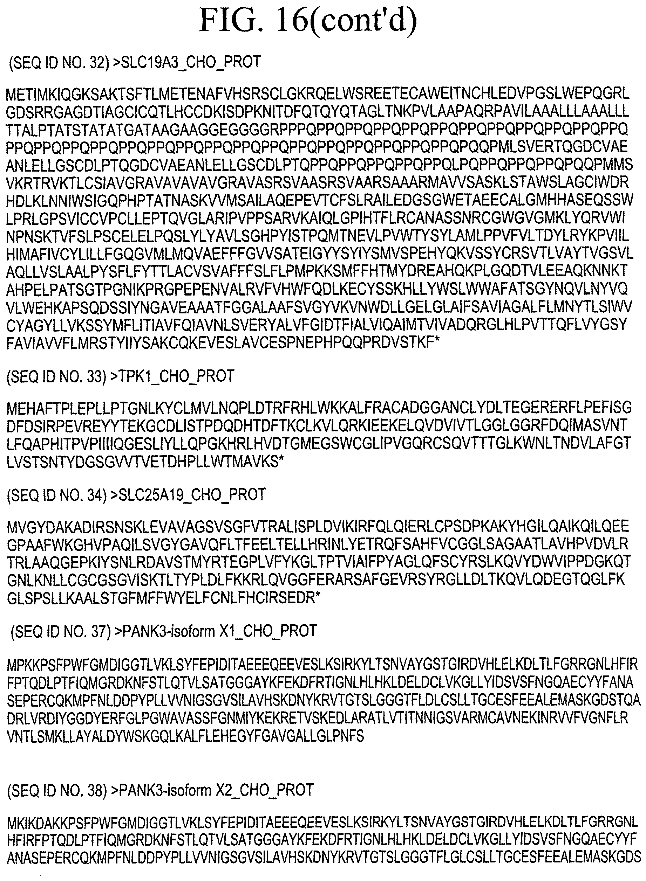

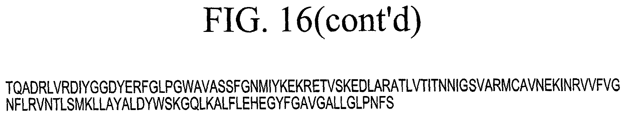

**Fig. 16** shows the amino acid sequences of the CHO-M vitamin genes of Fig. 15.

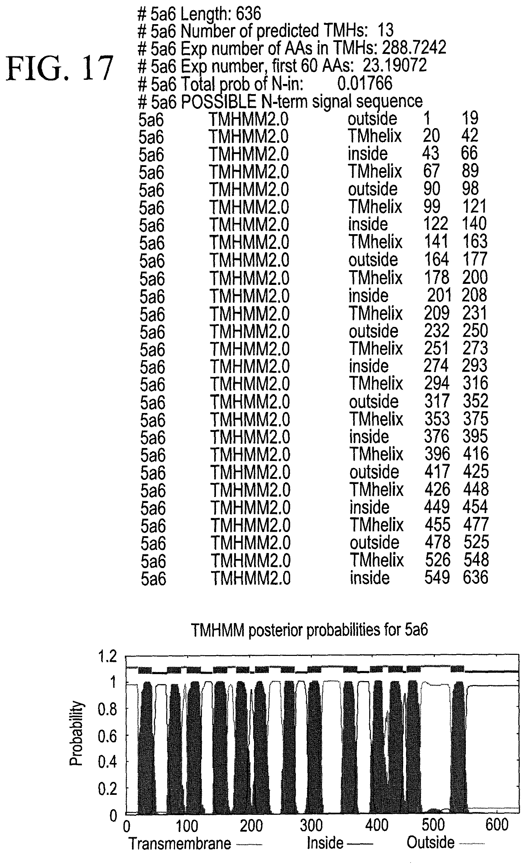

**Fig. 17** shows the SLC5A6 sodium-dependent multivitamin transporter (SMVT) in silico prediction for transmembrane regions (determined via the website of the Center for Biological Sequence Analysis, Technical University of Denmark, March 2015).

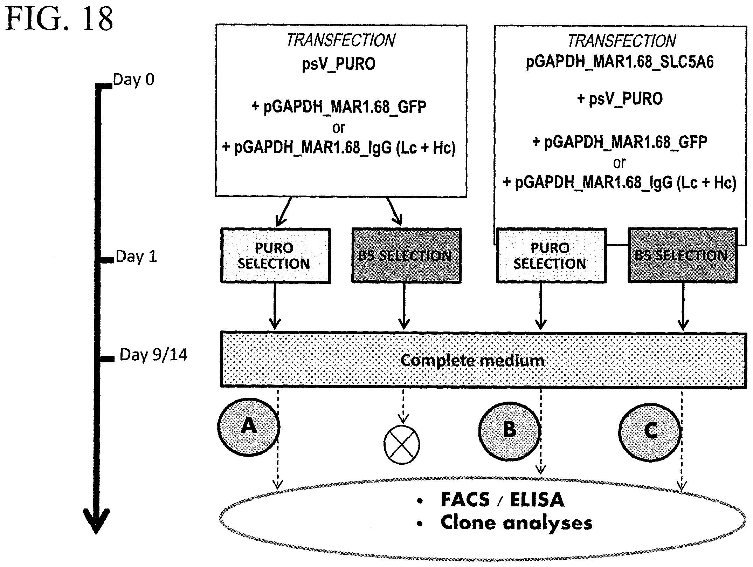

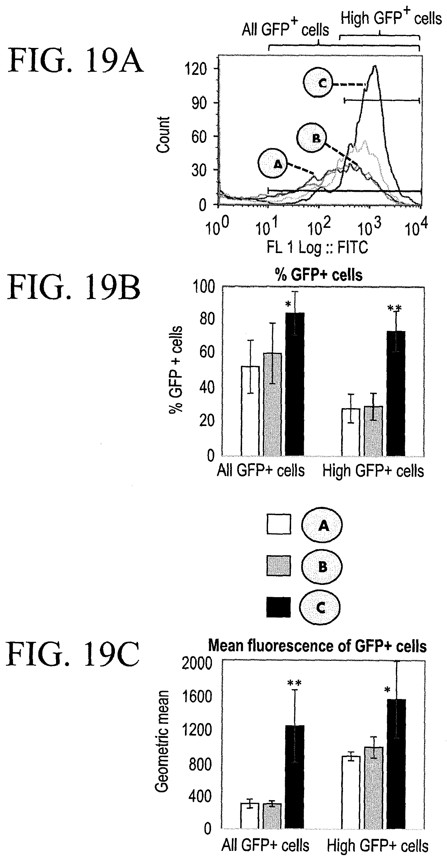

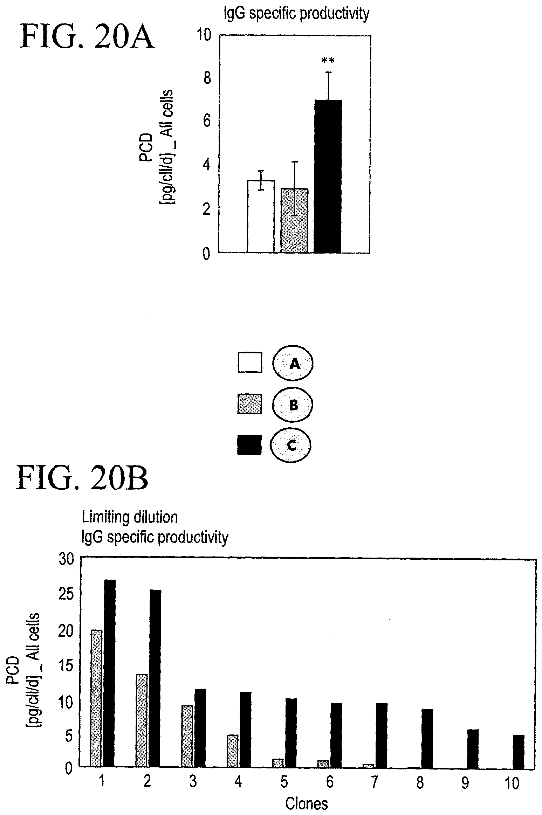

**Fig. 18** shows a protocol for selecting highly expressing cells by co-transfecting an expression vector for the SLC5a6 vitamin transporter (right) and culturing the cells in selective vitamin-deprived culture medium. CHO cells were co-transfected with the GFP or the IgG light and heavy chain expression vectors and the puromycin resistance plasmid, either without (condition A) or with (conditions B and C) the SLC5a6 expression vector. The cultures were then selected either in presence of puromycin (conditions A and B) or by culturing in the vitamin-deprived culture medium containing limiting (B5 10⁻³X / H 10⁻⁴X) vitamin concentrations (condition C). Note that the crossed circle indicates that cells that had not been transfected with the SLC5a6 expression vector did not survive selection in the vitamin-deprived culture medium. After selection, cells were cultured in a non-selective culture medium until analysis by FACS or by immunoglobulin secretion assays of the resulting polyclonal cell pools (Fig. 19-20), or during the generation and analysis of moncolnal populations (Fig. 20-21).
**Fig. 19** shows the enrichment of cells expressing the GFP reporter protein transfected according to the protocol shown in Fig. 18. Analysis by cytofluorometry for GFP fluorescence **(A)** showed high levels of polyclonal populations following vitamin-deprivation based selection (circled C). The enrichment of GFP-positive fluorescent cells **(B)** and the geometric mean of the GFP fluorescence of the cells (C) are represented for the polyclonal cell pools.
**Fig. 20** shows the enrichment of cells expressing a therapeutic immunoglobulin (rather than GFP) at high levels in polyclonal populations following vitamin-deprivation based selection, according to the protocols shown in Fig. 18. The production levels of cells selected by vitamin deprivation (labeled C) are higher at the polyclonal cell pool level (panel **A**), and for 10 randomly selected cells clones obtained by limiting dilutions (**panel B**) (see also the legend of Fig. 18).

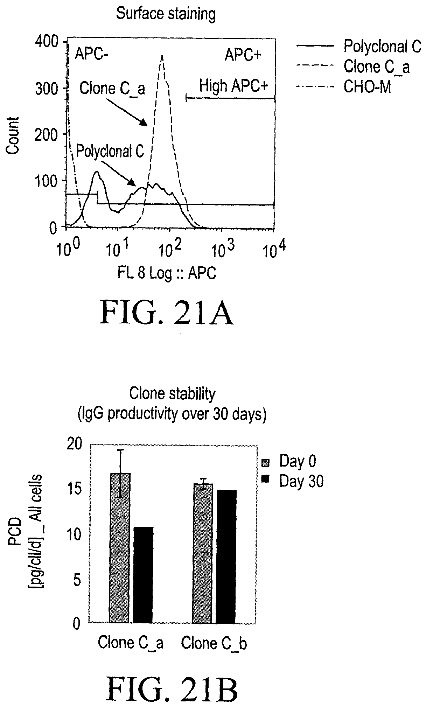

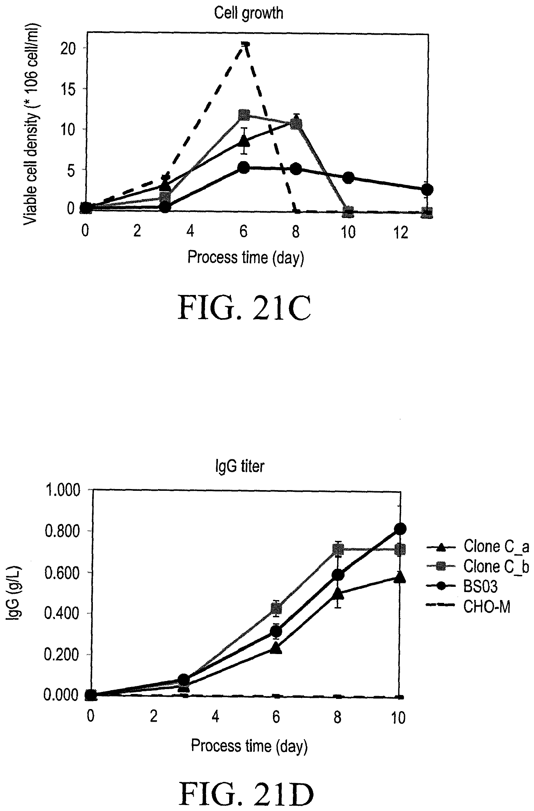

**Fig. 21** shows the high level of IgG secretion by cell surface staining for one of the IgG-producing clones (Clone C_a) obtained by vitamin selection **(A),** the stability of production for two such clones (Clones C_a and C_b) **(B),** as well as the high viable cell density and production levels of the two clones in fed-batch culture conditions (**C and D**), in comparison to a previously obtained high producer reference clone grown in parallel (BS03).

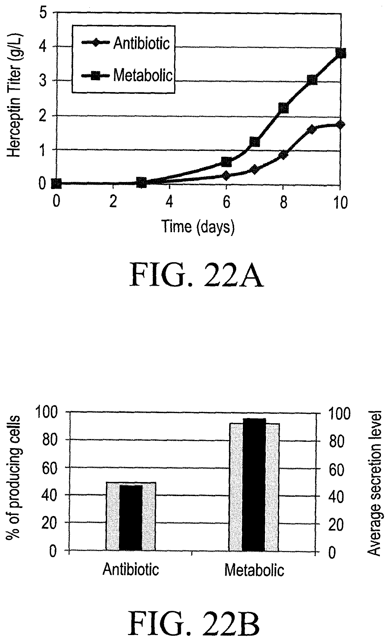

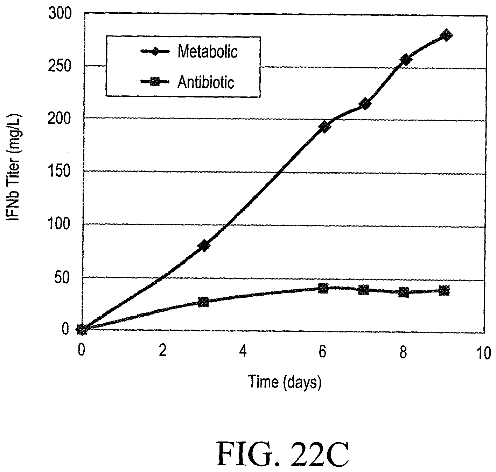

**Fig. 22** illustrates the selection (via an antibiotic or by culture in vitamin depleted medium ("metabolic")) of polyclonal populations expressing various therapeutic proteins, one easy-to-express antibody **(A and B)** and one difficult-to-express protein (interferon beta, panel C). This shows the versatility of the selection system for the selection of cells producing therapeutic proteins of interest at improved levels relative to conventional antibiotic selection.

### SUMMARY OF THE INVENTION

The invention is directed at a eukaryotic expression system comprising:
- at least one first polynucleotide encoding at least one vitamin metabolic protein under the control of at least one first regulatory sequence, and
- under the control of at least one second regulatory sequence, at least one restriction enzyme cleavage site and/or at least one second polynucleotide encoding at least one product of interest.

The at least one vitamin metabolic protein may be a vitamin transport protein. The at least one second polynucleotide may be inserted into said at least one restriction enzyme cleavage site. The vitamin transport protein may transport a soluble vitamin such as vitamin B1, B5 and/or H. The vitamin transport protein may be THTR-1 (thiamine transporter-1), THTR-2 (thiamine transporter-1), TPC (thiamine pyrophosphate Carrier), TPK (thiamine pyrophosphokinase) and/or, in particular SMVT (sodium dependent multi vitamin transporter). An expression vector may comprise the expression system. In particular, a singular vector may comprise said at least one first and said at least one second polynucleotide.

The first and/or second regulatory sequence may be promoters, enhancers, locus control regions (LCRs), matrix attachment regions (MARs), scaffold attachment regions (SARs), insulator elements and/or nuclear matrix-associating DNAs.

The invention is also directed at a kit comprising in one container, the eukaryotic expression system disclosed herein (in particular on one or more vectors) and, in a second container, instructions of how to use said system. The kit may further comprise a cell culture medium, preferably having a limiting and/or saturating concentration of at least one vitamin, such as of vitamin B1, B5 and/ or H.

The invention is also directed at a recombinant eukaryotic cell comprising the expression system described herein; and/or
having an up or down mutation in a vitamin metabolic protein, and a polynucleotide (second polynucleotide) encoding a product of interest, or a regulartory sequence regulating the expression of a polynucleotide encoding the vitamin metabolic protein, wherein the vitamin metabolic protein is optionally intrinsic to the cell. The cell may be a Chinese Hamster Ovary (CHO) cell. The at least one first polynucleotides may be mutated/ contain an up or down mutation. The vitamin metabolic protein may interfere with vitamin metabolism and/or bind the vitamin within a cell. The vitamin metabolic protein may be pantothenate 1, 2 and/or 3 and/or a thiamin pyrophosphate kinase, such as TPK1 (thiamin pyrophosphate kinase 1).

The vitamin metabolic protein may be a selectable marker for said recombinant eukaryotic cell and said recombinant eukaryotic cell may produce and, preferably secret said product of interest.

The invention is also directed at a eukaryotic cell culture medium comprising the recombinant eukaryotic cells disclosed herein, preferably polyclonal, preferably expressing (i) a vitamin transport protein as the selectable marker and (ii) a protein of interest. The medium may be a limiting medium for B5, or a saturated medium for B5 but a limiting medium for H.

The invention is also directed at a method for culturing and, optionally selecting recombinant eukaryotic cells comprising:
providing the expression system as disclosed herein,
providing eukaryotic cells, wherein viability, growth and/or division of said eukaryotic cells is dependent on vitamin uptake,
introducing said expression system into said eukaryotic cells to produce said recombinant eukaryotic cells expressing said vitamin metabolic protein and said protein of interest,
culturing said eukaryotic cells in a cell culture medium, e.g., a limiting medium for B5, or a saturated medium for B5 but a limiting medium or not limiting medium for H, or a saturated medium for H but a limiting medium or not limiting medium for B5, and
optionally, selecting via said vitamin metabolic protein, which is preferably expressed on the surface of said recombinant eukaryotic cells, said recombinant eukaryotic cells that stably express said product of interest.

A selection medium as disclosed herein might be a limiting medium for B5, or a saturated medium for B5 but a limiting or non-limiting medium for H.

The present invention is also directed at the use of a vitamin metabolic protein and it's DNA coding sequence as a selection marker for selection of recombinant eukaryotic cells stably expressing a product of interest, wherein viability, growth and/or division of said cell may be dependent on the uptake of a vitamin.

The present invention is also directed at a culture medium comprising at least one vitamin:
- in a concentration of less than 10nM, and/or
- in a concentration of 20µM or more, wherein said at least one vitamin is an essential vitamin.

The at least one vitamin may be vitamin B1, B5 and/ or H. The culture medium may comprise one or more recombinant eukaryotic cells expressing, preferably secreting, a protein of interest. The protein of interest may be a therapeutic protein. Growth and/or division of the cells may be arrested, and the protein of interest may be produced at a maximum arrested level (MAL in [g/1]) that exceeds a maximum level (ML in [g/1]) of protein expressed by the cells when grown in a medium, preferably a standard medium, in which growth is not arrested, wherein the MAL is more than 1,5 x the ML, more than 2 x the ML or even more than 2,5x or 3x the ML.

The invention is also directed at a method of producing a protein of interest, comprising:
(a) transforming eukaryotic cells with an expression system disclosed herein to produce recombinant eukaryotic cells;
(b) culturing said recombinant eukaryotic cells in a culture medium in which viability and/or growth or division of the recombinant eukaryotic cells is dependent upon activity of one or more vitamin metabolic protein;
(c) selecting for recombinant eukaryotic cells expressing said one or more vitamin metabolic protein, wherein said vitamin metabolic protein is a selectable marker to obtain selected recombinant eukaryotic cells, preferably when said recombinant eukaryotic cells are part of a monoclonal cell population (originating from a single cell); and
(d) purifying the protein of interest from said selected recombinant eukaryotic cells or from a culture medium thereof comprising said selected recombinant eukaryotic cells.

The vitamin metabolic protein may be a vitamin transport protein preferably transporting vitamin B5, B1 and/or H and said culture medium may be limiting and/or saturating for one of more of said vitamins. The vitamin transport protein may be SMVT and the culture medium may be a limiting medium for B5, or a saturated medium for B5 but a limiting medium for H.

The invention is also directed at cells, methods, systems and expression vectors disclosed herein, wherein said SMVT protein is encoded by a Slc5a6 gene or a derivative thereof, and/or wherein said eukaryotic cells are part of a monoclonal cell population.

The present invention is also more generally directed at assessing whether the strict vitamin requirements of eukaryotic cells could be used as selection tool for transformed cells, in particular transformed cells that stably express high levels of a gene of interest, when co-expressed with a vitamin uptake gene. The present invention is also more generally directed at assessing whether vitamin-depleted or enriched culture media may be used to further improve protein production by such cells.

In one specific embodiment, the present invention is directed at decreasing the availability of vitamin B5 at the late phase of recombinant protein production to slow cell division, and thereby to increase the level of therapeutic proteins produced in a bioreactor.

In one other specific embodiment, the invention is also directed at cloning and expressing the multivitamin transporter Slc5a6 (SMVT), involved in the uptake of both vitamin B5 and H into the cell, in particular CHO-M cells. The invention is also directed at cells overexpressing this vitamin transporter to result in faster growth and higher viability in B5-limiting media when compared to non-transformed cells. The invention is also directed at co-expressing SLC5A6 as a selection marker to obtain cell lines having higher levels of recombinant protein production. The invention is furthermore directed at overexpressing SLC5A6 in cells to produce better cell viability even in a non-depleted culture media, preferably contributing to even more favorable expression levels of therapeutic proteins.

### DISCUSSION OF VARIOUS AND PREFERRED EMBODIMENTS

### Definitions

A eukaryotic expression system according to the present invention comprises elements that allow for expression of a gene of interest in a eukaryotic cells such as a CHO cell, perferably a CHO K1 cell, preferably a CHO-M cell. Generally, the eukaryotic expression system comprises at least one expression vector. However, the eukaryotic expression system might also be part of the genome of a eukaryotic cell. The system/ expression vector comprises regulatory sequences such as promoters, enhancers, locus control regions (LCRs), matrix attachment regions (MARs), scaffold attachment regions (SARs), insulator elements and/or nuclear matrix-associating DNAs that lead to efficient transcription of a transgene integrated into the expression system. These regulatory sequences as any other sequences referred to herein are often heterologous (i.e., foreign to the host cell being utilized, e.g., derived from a different species as the host cell being utilized) or, while being homologous (i.e., endogenous to the host cell being utilized) are present at different genomic location(s) than any counterpart intrinsic to the cells (hereinafter referred to as "heterolocal"). An expression vector may also contain an origin of replication.

The first polynucleotide encoding at least one vitamin metabolic protein and the second polynucleotide encoding at least one product of interest according to the present invention are added to a eukaryotic cell to create a recombinant eukaryotic cell. Genes or proteins intrinsic to the eukaryotic cell are not added to the cell, but exist in the cell independent of any transformation. However, as the person skilled in the art will realize, the first and second polynucleotide might be copies of an intrinsic gene, such as heterolocal copies of the gene. In many instances it is preferred that some or all of the coding DNA sequences (CDSs) of a wild type gene make up the polynucleotides of the present invention, including the first polynucleotide encoding at least one vitamin metabolic protein.

As used herein, "plasmid" and "vector" are used interchangeably, as a plasmid is the most commonly used vector form. However, the invention is intended to include such other forms of expression vectors, including, but not limited to, viral vectors (e.g., replication defective retroviruses, adenoviruses and adeno-associated viruses), or transposable vectors, which serve equivalent functions. Herein, transformation refers to the introduction of vector DNA into any cell, irrespective the means or type of vector used.

The "gene of interest" or "transgene", herein also referred to as "polynucleotide encoding a product of interest" encodes, e.g., a "protein of interest" (structural or regulatory protein). The protein of interest is often a therapeutic protein. As used herein "protein" refers generally to peptides and polypeptides having more than about ten amino acids. The proteins may be "homologous" to the host (i.e., endogenous to the host cell being utilized), or "heterologous," (i.e., foreign to the host cell being utilized), such as a human protein produced by yeast. The protein may be produced as an insoluble aggregate or as a soluble protein in the periplasmic space or cytoplasm of the cell, or in the extracellular medium. Examples of therapeutic proteins include hormones such as growth hormone or erythropoietin (EPO), growth factors such as epidermal growth factor, analgesic substances like enkephalin, enzymes like chymotrypsin, receptors, or antibodies (e.g..Trastuzumab monoclonal immunoglobulin (IgG)). Genes usually used as a visualizing marker e.g. green fluorescent protein are also suitable transgenes. The transgene may also encode, e.g., a regulatory RNA, such as a siRNA. A homologous protein or RNA might be produced by a heterolocal polynucleotide. In many instances it is preferred that some or all of the coding DNA sequences (CDSs) of a wild type gene make up the polynucleotides of the present invention, including the second polynucleotide encoding at least one product of interest.

Eukaryotic cells used in the context of the present invention include, but are not limited to, the above mentioned CHO-M cells (available from SELEXIS SA), and other cells which are suitable for protein production at industrial manufacturing scale. Those cells are well known to the skilled person and have originated for example from Cricetulus griseus, Cercopithecus aethiops, Homo sapiens, Mesocricetus auratus, Mus musculus and Chlorocebus species. The respective cell lines are known as CHO-cells (Chinese Hamster Ovary), COS-cells (a cell line derived from monkey kidney (African green monkey), Vero-cells (kidney epithelial cells extracted from African green monkey), Hela-cells (The line was derived from cervical cancer cells taken from Henrietta Lacks), BHK-cells (baby hamster kidney cells, HEK-cells (Human Embryonic Kidney), NSO-cells (Murine myeloma cell line), C127-cells (nontumorigenic mouse cell line), PerC6.RTM.-cells (human cell line, Crucell), CAP-cells (CEVEC's Amniocyte Production) and Sp-2/0-cells (Mouse myeloma cells). Eucaryotic cells used in the context of the present invention may also, e.g., be human primary cells including hematopoietic stem cells, such as cells from bone marrow or stem cells, such as embryonic stem (ES) cells, induced pluripotent stem (iPS) cells or differentiated cells derived from ES or iPS cells.

A vitamin metabolic protein according to the present invention is a protein which either lowers or increases vitamin availability or use in a cell.

One preferred vitamin metabolic protein is a vitamin transport protein which is generally a membrane-bound protein and transports vitamins available in a culture medium into a cell. Table 1 provides examples of those proteins under the heading "Function". As can be seen from this table, two cytoplasmic and one mitochondrial transporters have been characterized for vitamin B1 (SLC19A2 [SEQ ID NO. 24], SLC19A3 [SEQ ID NO. 25] and SLC25A19 [SEQ ID NO. 27]), whereas a single cytoplasmic transporter has been characterized for both the B5 and H vitamins, called the sodium-multivitamin transporter SLC5A6 [SEQ ID NO. 21].

Other examples of vitamin metabolic proteins include pantothenate kinases 1, 2 or 3 encoded by the *PANK1* [SEQ ID NO. 22], *PANK2* [SEQ ID NO. 23], and *PANK3* [SEQ ID NO. 35, 36] gene and the TPK1 (thiamin pyrophosphate kinase 1), encoded by the *TPK1* gene [SEQ ID NO. 26]. Pantothenate kinases are key regulatory enzyme in the biosynthesis of coenzyme A (CoA), the homodimeric TPK1 protein catalyzes the conversion of thiamine to thiamine pyrophosphate. As the person skilled in the art will readily realize, other proteins that are involved in vitamin metabolism are also part of the present invention.

A cell growing in a complete culture medium will have all vitamins available at standard concentrations. Standard concentrations are referred to herein as 1X. Standard concentrations for B1, B5 and H (1X) were set at 7.5µM, 2.5µM and 0.5µM , respectively. B5 was determined to have for CHO cells a growth-limiting concentration range around 10⁻⁴X to 10⁻³X (0.25 to 2.5nM), whereas 10⁻²X and higher concentrations allowed normal culture growth. The limiting concentrations of B1 was determined to be for CHO cells between 10⁻⁵X (15pM) and 10⁻⁴X (150pM), whereas it was lower than 10⁻⁵X (5pM) for H. In a medium having limiting concentration (limiting medium or depleted medium) of said vitamin the concentration is less than 1X, e.g. 10⁻¹ X, 10⁻²X, 10⁻³X, 10⁻⁴X, 10⁻⁵X, relative to said standard concentration of the respective vitamin present in a complete medium (1 X). The concentration of a vitamin is considered saturating if the concentration exceeds that in a standard reference medium (also referred to herein as a "saturated medium") (e.g., 2 X, 3 X, 4 X, 5 X, or 10 X the amount found in a complete medium).

Cell culture media having a limiting and/or a saturating concentration of a vitamin are part of the present invention. E.g., the medium may be depleted with respect to one vitamin, but saturated with respect to another vitamin.

In a limiting medium the growth and/or division of said cells may be arrested, and a protein of interest may be produced at a maximum arrested level ("MAL" in [g/1]). The MAL may exceed a maximum level ("ML" in [g/l]) of protein expressed by the same type of cells when grown in a medium such as a standard medium, in which their growth is not arrested. In certain embodiments of the present invention, the MAL is more than 1,5 x the ML, more than 2 x the ML or even more than 2,5x or 3x the ML. For example, while a ML of protein of interest, such as an antibody that is expressed by recombinant cells, such as recombinant CHO cells in standard medium is about 1 g/l of IgG, the MAL of protein of interest, such as an antibody that is expressed by recombinant cells, such as recombinant CHO cells in standard medium is about 3 g/l of IgG or more.

The vitamin metabolic protein, including the vitamin transport protein, may be a full length wild type protein or may be mutated, including by point mutations, substitutions, insertions, additions and/or terminal or internal deletions or inversions. While a vitamin metabolic protein may, relative to a particular sequence, contain a mutation which has (i) activity corresponding to the wild type protein (neutral mutation), a vitamin metabolic/transport protein is referred to as mutated in the context of the present invention when the mutation causes an (ii) altered activity/stability compared to the wild type protein which includes increased activity ("up mutation") (by e.g. more than 10%, more than 20%, more than 30%, more than 40%, more than 50%, more than 60%, more than 70%, more than 80%, more than 90% or more than 100%) or decreased activity/stability ("down mutation") (by e.g. less than 10%, less than 20%, less than 30%, less than 40%, less than 50%, less than 60%, less than 70%, less than 80%, less than 90% , less than or by 100%). Whether or not a particular mutation is an up or down mutation can be readily assessed be standard assays available in the art. The mutated vitamin metabolic protein results from a mutation in the least one first polynucleotide encoding the vitamin metabolic protein. Similarly, a mutation in the sequence regulating the expression of said first polypeptide is called an up-mutation when the polypeptide encoded by the polynucleotide is expressed more or more stably (e.g., 10%, 20%, 30%, 40%, 50%, or more) than when the in a sequence regulating the expression of said first polypeptide does not comprise the mutation. A mutation in in a sequence regulating the expression of said first polypeptide is called a down mutation when the polypeptide encoded by the polynucleotide is expressed less or less stably than the first polynucleotide e.g., 10%, 20%, 30%, 40%, 50%, or less) than when the sequence regulating the expression of said first polypeptide does not comprise the mutation. Up-mutations in the sequences regulating the expression of the first polypeptide may also correspond to the addtion of a MAR, SAR, LCR and./or an insulator element in addition to the enhancer and promoter sequences in order to increase the expression level or stablity of the protein encoded by said polynucleotide.

The desired modifications or mutations in the polypeptide may be accomplished using any techniques known in the art. Recombinant DNA techniques for introducing such changes in a protein sequence are well known in the art. In certain embodiments, the modifications are made by site-directed mutagenesis of the polynucleotide encoding the protein or the sequence regulating (regulatory sequences as defined above) its expression. Other techniques for introducing mutations are discussed in Molecular Cloning: A Laboratory Manual, 2nd Ed., ed. by Sambrook, Fritsch, and Maniatis (Cold Spring Harbor Laboratory Press: 1989); the treatise, Methods in Enzymnology (Academic Press, Inc., N.Y.); Ausubel et al. Current Protocols in Molecular Biology (John Wiley & Sons, Inc., New York, 1999).

Well known are in particular down mutations in promoters and other regulatory sequences inherent in a cell. The mutation lowers the affinity of the transcription factors for the promoter region, lowering transcription rates. However, mutations in promoter regions may also be neutral or cause up mutations..

Polynucleotides and proteins having more than 50%, 60%, 70%, 75%, 80%, 85%, 90%, 95%, 96%, 97%, 98% or 99% sequence identity with the polynucleotides and proteins sequences disclosed herein, in particular those disclosed in Figs. 15 and16 are also part of the present invention either alone or as part of any system (e.g. vectors and cells), method and kit disclosed herein. Fig. 15 shows in particular the CDS (coding DNA sequence) of the respective gene, ergo that portion of the gene's DNA or RNA, composed of exons that codes for the respective protein / amino acid sequence (see Fig. 16). Polynucleotides of the present invention may differ from any wild type sequence by at least one, two, three, four five, six, seven, eight, nine or more nucleotides. In many instances, polynucleotides made up of CDSs of the respective gene or cDNAs are preferred.

The term sequence identity refers to a measure of the identity of nucleotide sequences or amino acid sequences. In general, the sequences are aligned so that the highest order match is obtained. "Identity", per se, has recognized meaning in the art and can be calculated using published techniques. (See, e.g.: Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H. & Lipton, D., SIAM J Applied Math 48:1073 (1988)).

Whether any particular nucleic acid molecule is at least 50%, 60%, 70%, 75%, 80%, 85%, 90%, 95%, 96%, 97%, 98% or 99% identical to, for instance, the SMTV nucleic acid sequence [SEQ ID NO. 21], or a part thereof, can be determined conventionally using known computer programs such as DNAsis software (Hitachi Software, San Bruno, Calif.) for initial sequence alignment followed by ESEE version 3.0 DNA/protein sequence software (cabot@trog.mbb.sfu.ca) for multiple sequence alignments.

Whether the amino acid sequence is at least 50%, 60%, 70%, 75%, 80%, 85%, 90%, 95%, 96%, 97%, 98% or 99% identical to, for instance SEQ ID NO. 28, or a part thereof, can be determined conventionally using known computer programs such the BESTFIT program (Wisconsin Sequence Analysis Package, Version 8 for Unix, Genetics Computer Group, University Research Park, 575 Science Drive, Madison, Wis. 53711). BESTFIT uses the local homology algorithm of Smith and Waterman, Advances in Applied Mathematics 2:482-489 (1981), to find the best segment of homology between two sequences.

When using DNAsis, ESEE, BESTFIT or any other sequence alignment program to determine whether a particular sequence is, for instance, 95% identical to a reference sequence according to the present invention, the parameters are set such that the percentage of identity is calculated over the full length of the reference nucleic acid or amino acid sequence and that gaps in homology of up to 5% of the total number of nucleotides in the reference sequence are allowed.

A recombinant eukaryotic cell according to the present invention is a eukaryotic cell containing a transgene as defined above.

An essential vitamin according to the present invention is a vitamin required for cell growth, division and/or viability.

Expression systems generally contain a selectable marker gene which facilitates the selection of eukaryotic cells (host cells) transformed with vectors containing the polynucleotide encoding the protein of interest. The selectable marker (or "selectable marker protein") expressed by the gene are often based on antibiotic resistance. E.g. a puromycin resistance selection expression cassette can be used to identify, via the addition of pyromycin, cells that has been successfully transformed with the cassette. However, selection without any resistance to antibiotics is also possible. Examples of selectable markers of this kind are dihydrofolate reductase (DHFR) and glutamine synthetase (GS). Selection occurs, e.g., in the absence of the metabolites e.g. glycine, hypoxanthine and thymidine for DHFR and glutamine for GS. Cells surviving selection comprise one or more copies of the transformed plasmid in the cell's genome. In the context of the present invention, the vitamin metabolic protein/ vitamin transport protein may serve as selectable marker either alone or in combination with other selectable markers. Thus, in its simplest form, in a medium that is deficient in one vitamin, recombinant eukaryotic cells expressing the respective vitamin transport protein as a selectable marker can grow better than cells not expressing the respective vitamin transport protein. However, as discussed herein, even in standard medium, the vitamin transport proteins provide a growth advantage and thus can be used as selectable marker. The expression systems of the present invention may contain, as selectable markers, vitamin metabolic protein(s)/ vitamin transport protein(s) in addition to selectable marker genes based, e.g., on antibiotic resistance.

Similarly, a mutation in the sequence regulating the expression of said first polypeptide is called an up-mutation when the polypeptide encoded by the polynucleotide is expressed more or is more stable (e.g., 10%, 20%, 30%, 40%, 50%, or more) than when the in a sequence regulating the expression of said first polypeptide does not comprise the mutation. A mutation in in a sequence regulating the expression of said first polypeptide is called a down-mutation when the polypeptide encoded by the polynucleotide is expressed less than the first polynucleotide or is less stable (e.g., 10%, 20%, 30%, 40%, 50%, or less) than when the sequence regulating the expression of said first polypeptide does not comprise the mutation.

The desired modifications or mutations in the polypeptide may be accomplished using any techniques known in the art. Recombinant DNA techniques for introducing such changes in a protein sequence are well known in the art. In certain embodiments, the modifications are made by site-directed mutagenesis of the polynucleotide encoding the protein or the sequence regulating (regulatory sequences as defined above) its expression. Other techniques for introducing mutations are discussed in Molecular Cloning: A Laboratory Manual, 2nd Ed., ed. by Sambrook, Fritsch, and Maniatis (Cold Spring Harbor Laboratory Press: 1989); the treatise, Methods in Enzymnology (Academic Press, Inc., N.Y.); Ausubel et al. Current Protocols in Molecular Biology (John Wiley & Sons, Inc., New York, 1999).

Well known are in particular down mutations in promoters and other regulatory sequences inherent in a cell. The mutation lowers the affinity of the transcription factors for the promoter region, lowering transcription rates. Mutations in promoter regions may be neutral, cause down or up mutations.. Similarly, mutations in, e.g., a gene for a vitamin metabolic protein such as a vitamin transport protein may be neutral, be down or up mutations.

### 1- Effects of limiting vitamin transport on CHO cell growth and recombinant protein expression

A first step to the use of vitamins by cultured mammalian cells is their cellular uptake from the culture medium. Vitamins B1 (thiamin), B5 (panthotenate) and H (B8 or biotin) are soluble vitamins that are transported in the cytoplasm and then into the mitochondria, where they act as metabolic cofactors (Fig.1). Two cytoplasmic and one mitochondrial transporters have been characterized for B1 (SLC19A2, SLC19A3 and SLC25A19), whereas a single cytoplasmic transporter has been characterized for both the B5 and H vitamins, called the sodium-multivitamin transporter SLC5A6 (Table1).

**Table 1. Mice genes involved in vitamin uptake into the cell. Last column: transcript accumulation in CHO-M cells.**

| **Vitamin** | **Function** | **Localization** | **Accession** | **CHO-M transcriptome (hit number in RPKM)** |
|---|---|---|---|---|
| B1 | (THTR)-1 (Thiamine Transporter) | Plasma membrane | S1c19a2 | 3270/12 |
| | (THTR)-2 (Thiamine Transporter) | Plasma membrane | S1c19a3 | 0/0 |
| | TPK (Thiamine Pyrophosphate Kinase) | Cytosol | Tpk1 | 4899 / 12 |
| | TPC (Thiamine Pyrophosphate Carrier) | Mitochondria | S1c25a19 | 6016/22 |
| H+ B5 | SMTV (Sodium-dpdt MulTiVitamin transporter) | Plasma membrane | Slc5a6 | 5267 / 13 |
| B5 | PANK1 (Pantothenate Kinase 1) | Mitochondria | Pank1 | 3 |
| | PANK2 (Pantothenate Kinase 2) | Mitochondria | Pank2 | 38 |

### 1.1- Determining the growth-limiting vitamin concentrations

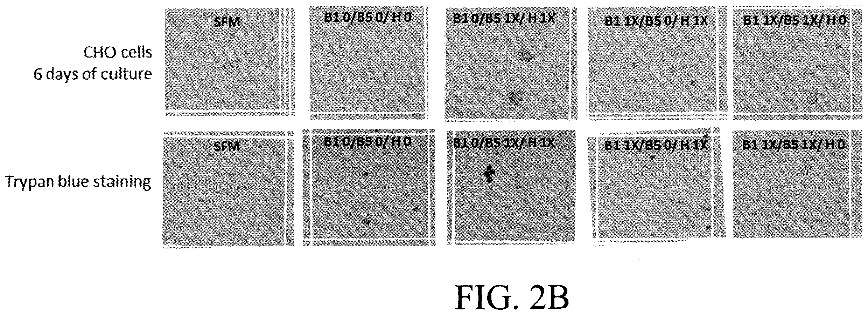

To assess the effect of limiting vitamin concentration on cell growth, a cell culture medium specifically depleted of vitamins B1, B5 and H, called B-CDmin, was derived from a commercially available growth medium (BalanCD CHO growth medium, IRVINE SCIENTIFIC INC). CHO-M cells seeded in the B-CDmin medium were unable to maintain cell divisions, as expected (Fig 2A). Over time, cell size was reduced, and the cells started to loose viability after 6 days of incubation in the vitamin-lacking medium (Fig. 2B). The B-CDmin medium was next complemented with known amounts of the vitamins, setting standard B1, B5 and H concentrations (1X) at 7.5µM, 2.5µM and 0.5µM, respectively, as found in commonly used complete media (Table 2). In the culture medium deficient solely of B5, cells did not divide and viability decreased after 6 days, as in the B-CDmin medium (Fig. 2A and 2B). When either B1 or H was depleted, cells were able to divide for 3 to 6 days respectively, although culture growth was reduced overall in the H-depleted medium as compared to the full media. Therefore, we concluded that B5 may be most limiting for cell growth in the short term, as it must be present continuously in the culture medium to maintain cell division.

**Table 2. Vitamin B1, B5 and H composition in SLX and CDM4CHO media (done by mass spectrometry, cf. Selexis), and concentration added in the BalanCD minimum media.**

| Culture media | B1/Thiamin | B5/ Panthotenate | H/Biotin |
|---|---|---|---|
| SLX medium | 8.84µM | 14.26µM | 90nM |
| CDM4CHO medium | 6.75µM | 2.99µM | 3.06µM |
| BalanCD minimum + vitamin B1, B5, H (1X) | 7.5µM | 2.51µM | 0.5µM |

The depleted B-CDmin medium was complemented with lower concentration of each vitamin separately, to determine the contrations range limiting CHO-M growth. B5 was essential for CHO-M growth, with a growth-limiting concentration range around 10⁻⁴X to 10⁻³X (0.25 to 2.5nM), whereas 10⁻²X and higher concentrations allowed normal culture growth (Fig 3 and data not shown). The limiting concentrations of B1 were observed between 10⁻⁵X (15pM) and 10⁻⁴X (150pM), whereas it was lower than 10⁻⁵X (5pM) for H. Interestingly, in presence of H at a low concentration (10⁻⁵X), the cell density was slightly higher than that observed in the full medium. As the B5 and H vitamins both use the same transporter to enter the cell, and because B5 is most limiting for cell growth, decreasing H concentration below saturating level might have increased the transporter availability for B5, which may allow B5 to reach higher intracellular levels as compared to cells grown in a full medium.

### 1.2- Effect of growth-limitine B5 vitamin concentration on protein expression and modifications

It was next assessed whether the growth arrest observed upon the depletion of B5 may be used to interrupt or slow down cell division in protein production conditions, so as to possibly increase protein production, using fed-batch cultures maintained in spin-tube bioreactors. A CHO-M derived cell clone expressing a therapeutic protein displayed an increase of the cell number until day 8 when grown in the complete medium, after which the cell viability and viable cell number dropped, as usually observed from these culture conditions (Fig. 4). However, in vitamin-limiting conditions, the cell number plateaued from day 7, and a high cell viability was maintained until day 14, indicating that the cells utilized the limiting vitamin availability from the medium and their endogenous cellular B5 pool for a limited number of cell divisions before it became a growth-limiting factor.

The titer of the antibody secreted in the cell culture supernatant increased up to 3g/L until day 9 in the complete medium culture, after which it declined (Fig. 5), as expected from the decreased cell viability noted earlier (Fig. 4). However, the antibody kept accumulating until day 15 of the culture performed with the vitamin depleted medium, where it reached levels over 6g/L (Fig. 5). Overall, we concluded that vitamin deprivation can be used to arrest the growth of cells in the bioreactor, so as to extend the longevity of cell viability and antibody secretion, thus providing very high titers of the therapeutic antibody. This approach may be generally applicable to improve recombinant protein production.

### 1.3- Effect of increasing B5 vitamin transport on cell growth

Based on the findings that cell growth can be inhibited either by the lack of B5, by high concentrations of H, which can compete with B5 for their common transporter, or by high concentrations of B5, which can compete with H for their common transporter, it was hypothesized that overexpressing the common Slc5a6 transporter might provide a growth advantage to the cells and/or may lead to higher viable cell densities. We thus cloned the CHO-M cDNA encoding the multivitamin Slc5a6 transporter, and other vitamin B1 transporters, as indicated in Table 1, and inserted them under the control of the strong *GAPDH* promoter and MAR 1-68 epigenetic activator element, next to a puromycin resistance selection expression cassette. CHO-M cells were co-transformed with this Slc5a6 construct, with a GFP expression vector and with a puromycin selection plasmid, after which stable polyclonal populations were obtained from the selection of puromycin-resistant cells. Up to 100-fold higher Slc5a6 transcript accumulation was observed in populations of CHO-M cells transformed with increasing amounts of the expression vector, when compared to the endogenous expression level (Fig. 6).

Cell populations overexpressing *SLC5A6* were then grown without puromycin selection in the B-CDmin medium supplemented with various concentrations of B5 and H. As before, cell division nearly arrested in the absence of B5 after 6 days of culture, irrespective of the overexpression of the transporter or of the presence of vitamin H (Fig. 7). However, cells transformed with the transporter expression plasmid reached significantly higher densities in limiting condition of B5 (10⁻³X) and with low H (10⁻⁴X). The highest growth was observed from the cells co-transformed with 100ng of the transporter expression vector (Fig. 7), suggesting that an optimal expression level of the transporter was achieved.

Interestingly, when B5 was added in 10X excess in presence of the low H amount (10X B5; 10⁻⁴X H), untransformed cell growth was strongly inhibited relative to the culture of these cells in the complete medium (1X B5; 10⁻⁴X H). However, cells expressing the highest transporter level grew significantly more than those expressing the transporter at lower levels in the presence of the excess of B5 (10X B5; 10⁻⁴X H). This further indicated the occurrence of a competition of the two vitamins for their common transporter, where saturating concentrations of B5 may inhibit the uptake of low amounts of H in the culture medium, thus limiting growth, unless the transporter is overexpressed. Overall, it was concluded that overexpression of the SLC5A6 transporter can confer a growth advantage in presence of either limiting concentrations of B5, or conversely in presence of saturating concentrations of B5 but with limiting amounts of H. It was hypothesized that this might therefore be used to discriminate cells that express elevated amounts of the transporter against those that express it at lower levels.

### 2- Use of SLC5A6 (SMVT) transporter expression as a selection marker for transformed cells

The expression from the co-transformed GFP vector was quantified to determine if the co-transformation of the Slc5a6 transporter may have increased the overall transgene expression levels. Cells having integrated the plasmids in their genome and stably expressing the transgenes were selected either by culture in a B5-limiting medium or in the presence of puromycin. The percentage of GFP-expressing fluorescent cells as well as the cellular fluorescence intensities were first assessed following selection by B5 deprivation. Upon selection in presence of limiting amounts of B5 (10⁻³X), the highest proportion of both the GFP-positive cells and the average fluorescence levels were obtained when co-transforming the cells with 250ng of the *SLC5A6* expression plasmid (Fig. 8). Transformation of higher plasmid amount (1000ng) of the Slc5a6 vector gave similar numbers of GFP-positive cells and slightly lower fluorescence, whereas lower plasmid amount (50ng) did not yield enough cells for quantification. This indicated that the co-transformation of this vitamin transporter gene can be used as a selectable marker for stable transformation, by co-transforming a small amount of the *SLC5A6* plasmid with higher amounts of a construct expressing a protein of interest (Table 3). A small amount of the *SLC5A6* plasmid is typically 1000ng, 250 ng, 100ng or less (see, e.g., Figs 6, 8 and 9). As illustrated in Table 3, higher amounts of a construct expressing a protein of interest may range from more than twice to more than 15 times the amount of the vitamin metabolic protein expression vector, including more than 3, 4, 5, 6, 7, 8, 9, 10, 11, 12, 13 or 14 times. Less favorable results were obtained when similar experiments were performed with the vitamin B1 transport cDNAs (data not shown), as might be expected from the fact that B5 is more limiting than vitamin B1.

**Table 3. Vector mixes used for transfections with Slc5a6+puro+GFP/IgG**

| **Vectors** | **pGAPDH-MAR1-68-Slc5a6 (10148bp)** | **pGAPDH-MAR1-68-GFP (8978bp)** | **pSV-Puro (4118bp)** | **pGAPDH-1.68-IgG_{Lc} (12551bp)** | **pGAPDH-1.68-IgG_{Hc} (13274bp)** |
|---|---|---|---|---|---|
| **Mix1: GFP/puro (ng)** | 0 | 1884 | 23 | 0 | 0 |
| **Mix2: Slc5a6(50ng)/GFP/puro (ng)** | 50 | 1834 | 23 | 0 | 0 |
| **Mix3: Slc5a6(100ng)/GFP/puro (ng)** | 100 | 1784 | 23 | 0 | 0 |
| **Mix4: Slc5a6(250ng)/GFP/puro (ng)** | 250 | 1634 | 23 | 0 | 0 |
| **Mix5: IgG_{Lc}/IgG_{Hc}/puro (ng)** | 0 | 0 | 17.9 | 1000 | 769.2 |
| **Mix6: Slc5a6(250ng)/IgG_{Lc}/IgG_{Hc}/pur o (ng)** | 250 | 0 | 17.9 | 1000 | 769.2 |

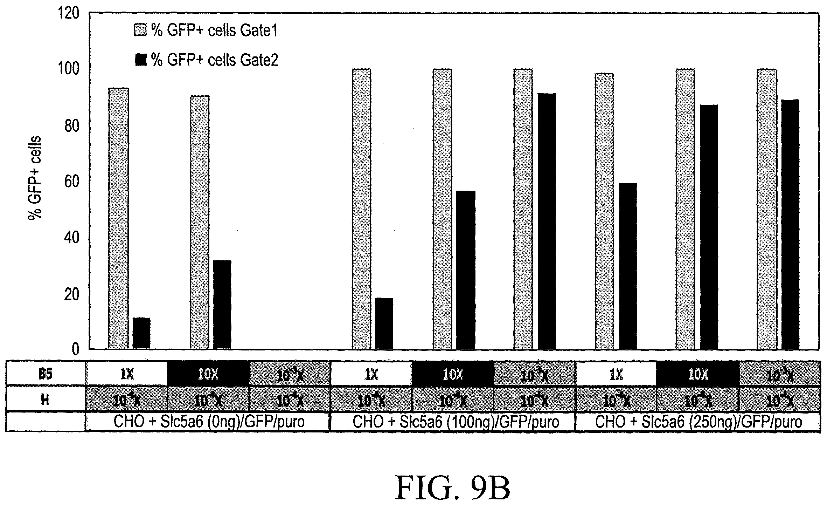

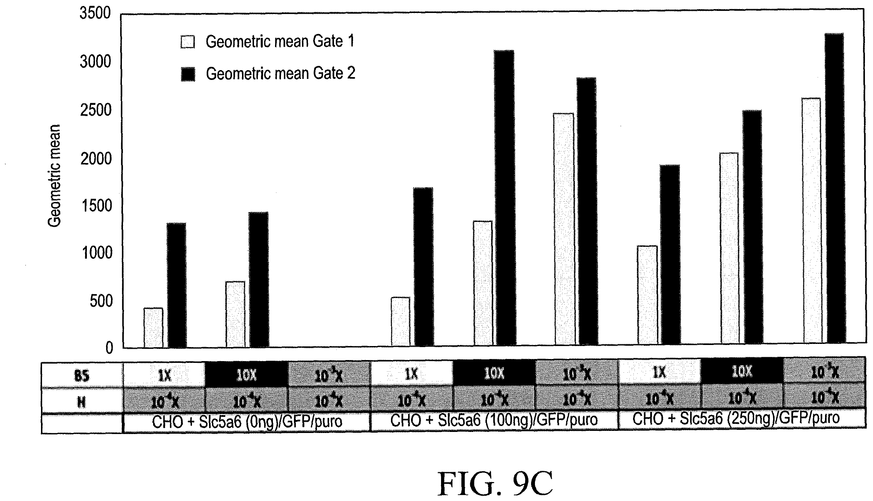

When the cells were selected by puromycin in a medium containing a non-limiting B5 concentration, GFP fluorescent cells were obtained irrespective of Slc5a6 expression, as expected. Nevertheless, the most highly fluorescent cells were often obtained upon the co-transformation of 250ng of the Slc5a6 expression vector (data not shown). This indicated that the vitamin transporter may confer a selective advantage to cells that express it at higher levels even in non-limiting culture media. When puromycin selection was followed by further culture in the vitamin B5-limiting medium, extremely high expression levels were observed in most of the cells overexpressing the SLC5a6 transporter (Fig. 9A). Quantification of the total percentile of GFP-expressing cells (Gate 1 of Fig. 9A), or of highly expressing cells (Gate 2 of Fig. 9A), revealed that over 80% of the cells expressed GFP at very high levels following the transformation of 100 or 250 ng of the Slc5a6 expression plasmid, either when selecting the cells in B5-depleted medium or in an excess of B5 (Fig. 9B). The GFP expression levels were also increased more than two-fold when vitamin B5 selection was performed following puromycin selection, as compared to performing a puromycin selection only (compare 0ng Slc5a6 and 1XB5, 10⁻⁴X H with 100 or 250ng *SLC5A6* and 10X B5; 10⁻⁴X H or 10⁻³X B5; 10⁻⁴X H, Fig. 9C). Overall, this indicated that Slc5a6 and vitamin-mediated selection can also be used in conjunction with antibiotic selection to select preferentially the cells that mediate the highest transgene expression levels.

This approach was pursued for the expression of a transgene encoding a therapeutic recombinant protein, namely the Trastuzumab monoclonal immunoglobulin (IgG). Cells were co-transformed with a plasmid encoding both Slc5a6 and the immunoglobulin light chain, and with another vector expressing the puromycin resistance marker and the immunoglobulin heavy chain. Cells were then selected under various regimen of B5 deprivation or puromycin treatment (Fig. 10), and the secreted Trastuzumab IgG was detected by cell-surface staining using a fluorescent anti-IgG antibody.

It was first assessed which of the selection conditions yielded polyclonal cell populations displaying the highest IgG secretion levels in the supernatants of fed batch cultures. Cells selected with puromycin only yielded the lowest levels of secreted IgG (A+ condition, Fig. 11). Cells selected by vitamin B5 deprivation (B1+), or by vitamin deprivation followed by the addition of puromycin in the minimal medium (B1D+ and B2D+), yielded comparably high IgG levels. Cell selected with puromycin followed by vitamin B5 deprivation yielded intermediate IgG titers. Given that performing puromycin selection in addition to vitamin depletion did not yield a significant increase relative to the selection with just B5 deprivation (Fig. 11, compare B1D+ with B1+), the next focus was on the analysis of the cells selected by vitamin deprivation only, using puromycin-selected cells as controls.

The highest proportion of IgG-expressing cells, in the 80 to 90% range, and the most elevated levels of cell surface fluorescence, were observed for the polyclonal cell pools selected using vitamin deprivation (Fig. 12A, B+ condition). High and yet balanced levels of the IgG heavy and light chain mRNAs were obtained upon vitamin B5 selection, and the mRNA levels of the Slc5a6 transporter expressed for selection purposes was found to be quite low relative to those of the IgG (Fig. 12B). The IgG secretion rates were found to be approximately 3-fold higher for the polyclonal populations selected by vitamin deprivation when compared to antibiotic selection, and immunoglobulin expression was found to be stable upon extended culture in the non-selective complete medium, even when it was secreted at the highest levels (Fig. 13). When these polyclonal cell populations were assessed in fed batch cultures using the complete culture medium, titers exceeding 8g/L were obtained for the populations selected by vitamin deprivation, whereas the titer obtained from the puromycin selection was at 2g/L (Fig. 14). Thus, the vitamin-deprivation and SLC5a6 overexpression-based selection of polyclonal populations yielded exceptionally high protein titers, in a range of IgG accumulation that is only occasionally obtained after the tedious and time-consuming sorting and selection of the most productive monoclonal populations.

An example of a process of cell selection is depicted in Fig. 18. CHO cells were co-transfected without (condition A) or with (conditions B and C) the SLC5a6 expression vector, together with the GFP or IgG light/heavy chain plasmids and the puromycin resistance, after which the culture was selected either in presence of puromycin (conditions A and B) or in the vitamin-deprived culture medium containing limiting (B5 10⁻³X / H 10⁻⁴X) vitamin concentrations (condition C). The crossed circle indicates that cells that had not been transfected with the SLC5a6 expression vector did not survive selection in the vitamin-deprived culture medium. As can be seen, the GFP plasmid used here contained also a MAR sequence. After selection, cells were cultured in a non-selective culture medium until analysis by FACS or immunoglobulin secretion assays of the resulting polyclonal cell pools (Fig. 19-20), or during the generation and analysis of monclonal populations (Fig. 20-21).

The GFP expressing polyclonal cell populations obtained in the process depicted in Fig. 18 were cultivated for 9 days in non-selective medium and were analyzed (Fig. 19). The analysis by cytofluorometry for GFP fluorescence provided FACS fluorescence profiles representing the enrichment of all GFP+ and of high GFP-expressing cells from stable polyclonal cell populations co-transfected with the Slc5a6, GFP and puromycin resistance (puro) expression plasmids, and selected by culturing with puromycin (conditions A and B, see Fig. 18) or in the vitamin-deprived culture medium (condition C). The proportion of cells and average fluorescence of all GFP-positive cells, and of the highly fluorescent cells, were determined from cells gated as illustrated in panel A of Fig. 19. The enrichment of GFP-positive fluorescent cells is shown in B of Fig. 19 and the geometric mean of the GFP fluorescence of the cells are represented for the polyclonal cell pools (Fig. 19C). As can be seen, B5 selection of cells transfected with the SLC5a6 expression vector, provided significant enrichment of GFP fluorescent cells among the high GFP-expressing cells (Fig. 19B) and significant increased geometric mean of the GFP fluorescence.

In Fig. 20, the immunoglobulin specific productivity of cell populations selected using puromycin or vitamin deprivation are shown. In Fig. 20A, the total polyclonal pools of cells expressing a therapeutic IgG were obtained as depicted for conditions A, B and C of Fig. 18, and the specific productivity of the IgG was assayed. The specific productivity is shown in picogram of secreted antibody per cell and per day (PCD) (Data are the results of three independant biological experiments. Two stars: P<0.05, one-sided, equal variance T-test). Fig. 20B shows the results obtained with ten clones that were randomly isolated by limiting dilutions of the cell populations obtained from selection conditions B and C, and the IgG specific productivity was determined. Again the specific productivity of cells cultures under condition C was significantly higher than the specific productivity of cells cultures under condition B.

Selected cell clones were further analyzed. In particular, two clones (C_a and C_b) obtained by the limiting dilution of a polyclonal cell pool expressing SLC5A6 and a therapeutic IgG, and selected using vitamin deprivation (Condition C in Fig. 18 and 20), were analyzed. The secreted IgG displayed at the cell surface was labelled by incubation with an IgG-directed fluorescent antibody, and cells were analyzed by cytofluorometry as shown in Fig. 21A. The fluorescence profiles of the initial polyclonal cell pool C and of the derived clone C_a are shown for comparison. In Fig. 21B, immunoglobulin expression stability of two monoclonal populations selected using vitamin depletion is depicted. The C_a and C_b clones were maintained in complete non-selective medium and passaged twice a week for 30 days. The specific productivity of the cell populations, expressed in picogram of secreted antibody per cell and per day (PCD), was assayed at the indicated time. As can be seen, the clones showed a high stability (PCD levels decrease not more than 50%, not more than 40%, not more than 30% not more than 20% or even not more than 10% or 5% from the original level when maintained in complete non-selective medium and passaged twice a week for 30 days). Fig. 21C and D show immunoglobulin production assays of fed-batch cultures of clones C_a and C_b. The clones were grown in complete medium in fed-batch cultures, and feeds were added at day 3, 6, 8 and 10. Samples were analyzed for viable cell density (Fig. 21C) and for the titer of secreted antibody by double sandwich ELISA (Fig. 21D). The high-IgG expressing BS03 clone and non-transfected parental CHO-M cells were used as reference. As can be seen, both clones performed well relative to the high-IgG expressing BS03 clone.

Figure 22 is an illustration of the selection of cell populations producing various recombinant proteins at high levels by SLC5a6 co-transfection and selection by vitamin deprivation. The titers obtained from fed-batch cultures of polyclonal populations of cells expressing an easy-to-express IgG, namely Herceptin, following either puromycin selection ("antibiotic") or selection by culture in vitamin-depleted medium ("metabolic") are shown in Fig. 22A. In Fig. 22B, the determination of the percentage of Herceptin expressing cells as well as the average secretion levels by colony imaging is shown. Titers obtained from polyclonal cell populations producing a difficult-to-express protein, namely Interferon beta, as selected by antibiotic addition or vitamin deprivation, are shown in Fig. 22C. As can be seen, especially the titers obtained from polyclonal cell populations producing the difficult-to-express protein, here Interferon beta, selected by vitamin deprivation exceeded those selected by antibiotic addition by 3 to 5 times.

It will be apparent to someone skilled in the art that other vitamin metabolic genes can be overexpressed for similar purposes, as depicted for instance in Fig. 1 and Table 1. The results obtained with B5 and Slc5a6 are shown here as examples, whereas the use of transporters for other vitamins (e.g. B1), or the use of other vitamin B5 metabolic genes (e.g. PANK, see Table 1), is also possible and within the scope of the present invention.

Similarly, host cells can be engineered to express lower levels of the transporter and other genes, to generate cell lines with even stronger selection properties. Finally, the use of cell culture media deprived of vitamins B1, B5 or H, or combinations thereof, as used in this study, is a general approach that can be used to increase the production levels of cells, whether they are engineered to overexpress one or more vitamin metabolic genes, as in Fig. 14, but also when using cells that are not modified in the expression levels of vitamin genes, as exemplified in Fig. 5. It will also be apparent to someone skilled in the art, and within the scope of the present invention, that this approach can be used to produce high levels of a therapeutic protein in vitro using cultured cell lines such as CHO-M cells, e.g. in a bioreactor, but also in vivo using primary cells such as human cells for gene or cell-based therapies, and also for regenerative medicine.

The above shows that polyclonal or monoclonal populations of cells producing recombinants proteins at homogeneous and very high levels can be obtained using coding sequences expressing vitamin metabolic proteins as selection markers. It was shown that vitamin deprivation during fed-batch bioreactor production conditions can be used to improve the viability of cell clones and their productivity in terms of the titer of secreted recombinant therapeutic proteins. Interestingly, these effects were obtained by lowering the levels of e.g. the B5 or H vitamins, but also when levels of one of the vitamins was raised above saturating levels. This later effect was noted when the elevation of B5 concentration above usual levels allowed the selection of cells that express high levels of the SLC5a6 selection gene, when grown in presence of low amounts of vitamin H. Thus, optimal selection regimen can also be designed by the increase of vitamin concentration, or by varying the relative levels of two vitamins that use the same membrane transporter. The approach described here is thus of high value for selecting and identifying cell clones that produce a protein of interest to more elevated and stable levels, and thus using reduced screening time and efforts, and also to increase protein production levels and cell viability independently of cell origin or vitamin gene engineering.

### Material and Methods

### Vitamin gene sequences and DNA vector constructs

Vitamin genomic and cDNA sequences were determined after alignment of the homologous genes in mice *SCL5A6, SLC19A2, SLC19A3, TPK1, SLC25A19* using NCBI BLAST software. Transcript sequence and accumulation of the corresponding genes was determined using SELEXIS CHO-M gene expression database. CDSs (coding DNA sequences) and protein sequences are listed in Fig. 15 and Fig. 16, respectively.

CHO-M (SURE CHO-M Cell Line™ (SELEXIS Inc., San Francisco, USA)), cDNA library was amplified by reverse transcription from 1ug total RNA isolated from 10⁶ CHO-M cells (NucleoSpin™ RNA kit; Macherey-Nagel) using Superscript Reverse Transcription Enzyme II and random primers (Goscript Reverse Transcription System; PROMEGA).

Vitamin coding sequences (CDS) were cloned into the *pGAPDH-MAR 1-68-GFP* vector, by cutting out the green fluorescent protein (*GFP*) gene and replacing it with the vitamin CDS. Vectors were constructed as follow: The CDS were amplified from CHO-M cDNA library by PCR (PHUSION High-Fidelity DNA Polymerase; Finnzymes, THERMO FISHER SCIENTIFIC) from ATG to Stop using primers carrying restriction site *HinIII*/*XbaI* for *SCL5A6, HinIII*/*FseI* for *SLC19A2, NcoI*/*XbaI* for *SLC19A3, HinIII*/*XbaI* for *TPK1, HinIII*/*XbaI* for *SLC25A19* (Table 4). Then, the cDNA products and pGAPDH vectors were double-digested by the corresponding restriction enzymes. Finally, the cDNAs were ligated into the *pGAPDH-MAR 1-68* vector where the GFP sequence was cut out after digestions with the same restriction enzymes.

**Table 4. Primer Sequences**

| PRIMER NAME | PRIMER SEQUENCE 5'_3' | PURPOSE | SEQ. ID. NO. |
|---|---|---|---|
| Slc5a6-ATG-HindIII_F | AAAAAGCTTATGAGTGTGGAAGAGAGCA | Cloning of the CDS | SEQ ID 1 |
| Slc5a6-Stop-Xba1_R | AAATCTAGATCACAGGGAGGTCTCCT | Cloning of the CDS | SEQ ID 2 |
| Pank2-ATG-HindIII_F | AAAAAGCTTATGTCTGGTGGCTTCCCTAAGG C | Cloning of the CDS | SEQ ID 3 |
| Pak2-Stop-Xba1_R | AAATCTAGATCACAACCGGTCAGC | Cloning of the CDS | SEQ ID 4 |
| Slc19a2-ATG-HindIII_F | AAAAAGCTTATGCATGGATTATGCAGCC | Cloning of the CDS | SEQ ID 5 |
| Slc19a2-Stop-FseI_R | AAAGGCCGGCCTTAGGGAGTAGTTGCTTGA | Cloning of the CDS | SEQ ID 6 |
| Slc19a3-ATG-Nco1_F | AAACCATGGAAACCATAATGAAGATA | Cloning of the CDS | SEQ ID 7 |
| Slc19a3-Stop-Xba1_R | AAATCTAGATCAGAACTTGGTTGACACAT | Cloning of the CDS | SEQ ID 8 |
| Tpk1-ATG-HindIII_F | AAAAAGCTTATGGAGCATGCGTTTACC | Cloning of the CDS | SEQ ID 9 |
| Tpk1-Stop-Xba1_R | AAATCTAGATTAGCTTTTGACGGCCATG | Cloning of the CDS | SEQ ID 10 |
| S1c25a19-ATG-HindIII_F | AAAAAGCTTATGGTCGGCTATGACGC | Cloning of the CDS | SEQ ID 11 |
| Slc25a19-Stop-Xba1_R | AAATCTAGACTATCTGTCTTCACTCCTTA | Cloning of the CDS | SEQ ID 12 |
| Slc5a6-qRT-F | GTGCCTATGAGTACCTGGAGCTT | Quantitative PCR | SEQ ID 13 |
| Slc5a6-qRT-R | AGCAACTCCCATGTAGATCACC | Quantitative PCR | SEQ ID 14 |
| IgG1-Lc-qRT-F | AGGACAGCAAGGACTCCACCTA | Quantitative PCR | SEQ ID 15 |
| IgG1-Lc-qRT-R | CGTACACCTTGTGCTTCTCGTAG | Quantitative PCR | SEQ ID 16 |
| IgG1-Hc-qRT-F | GGACCCTGAGGTGAAGTTCAAT | Quantitative PCR | SEQ ID 17 |
| IgG1-Hc-qRT-R | GGTAGGTGCTGTTGTACTGTTCC | Quantitative PCR | SEQ ID 18 |
| GFP-qRT-F | ACATTATGCCGGACAAAGCC | Quantitative PCR | SEQ ID 19 |
| GFP-qRT-R | TTGTTTGGTAATGATCAGCAAGTTG | Quantitative PCR | SEQ ID 20 |
| GAPDH-qRT-F | Quantitative PCR | | |
| GAPDH-qRT-R | Quantitative PCR | | |

The *pGAPDH-MAR 1-68-GFP* vector was described previously (Girod et al., 2007; Hart and Laemmli, 1998; Grandjean et al., 2011). The GFP protein was expressed using a eukaryotic expression cassette composed of a human cytomegalovirus (CMV) enhancer and human glyceraldehydes 3-phosphate dehydrogenase (GAPDH) promoter upstream of the coding sequence followed by a simian virus 40 (SV40) polyadenylation signal, the human gastrin terminator and a SV40 enhancer (Le Fourn et al., 2013).

The *pSV-puro* vector contains the puromycin resistance gene (puro) under the control of the SV40 promoter originated from pRc/RSVplasmid (INVITROGEN/LIFE TECHNOLOGIES).

The immunoglobulin expression vectors *1-68 filled-IgG1-Lc* and *1-68 filled-IgG1-Hc* were as previously described.

### Cell Culture, stable transformation and stable polyclonal line analyses

Suspension Chinese hamster ovary cells (CHO-M) were maintained in suspension culture in SFM4CHO-M Hyclone serum-free medium (SFM, ThermoScientific™) supplemented with L-glutamine (PAA, Austria) and HT supplement (GIBCO, INVITROGEN LIFE SCIENCES) at 37µC, 5% CO2 in humidified air. Other cell media used for these experiments are the BalanCD CHO-M Growth A (B-CDfull; Irvine Scientific), and the Deficient BalanCD CHO-M Growth A (B-CDmin; Irvine Scientific), supplemented with vitamin B1 (thiamine Hydrochloride; SIGMA ALDRICH), vitamin B5 (Calcium DL-Pantothenate; TCI) and vitamin H (Biotin, SIGMA ALDRICH)

CHO-M cells were transformed with *Pvul*-digested SLC5A6, GFP, puromycin, IgG1-Hc or IgG1-Lc expression vectors (see vector mixes in Table 3) by electroporation according to the manufacturer's recommendations (NEONDEVICES, INVITROGEN).

GFP and IgG1-producing cell polyclonal lines expressing the Slc5a6 and GFP or IgG were selected for further experiments as follow: One day before transformation, cells were grown at 300 000 cells/ml in B5 selective media which consisted in B-CDmin media supplemented with 7.5µM B1 (1X), 250nM B5 (10^{- 3}X) and 5uM H (10⁻⁴X). After transformation, cells were directly incubated in a 24-well plate with B5 selective media for 24h, then transferred to several wells depending on the experiments. For puromycin selection, cells were seeded in SFM media supplemented with 10mg/ml puromycin for 2 weeks, then transferred into well with SFM media for 5 days, then into 50ml spin tubes with SFM media.

For B5 selection, cells were seeded in B5 selective media for 7-9 days, then transferred into SFM non selective media as for puromycin selection.

For double selection of the cells with puromycin then B5, polyclonal stable cell lines were first selected with puromycin, then cells were seeded at 20 000 cells/ml in 24-well plate in B5 selective media for 7 days (B-CDfull media was used as negative control), then transferred in SFM full media wells for 7 days, then seeded into pin tube with SFM media.

The percentage of fluorescent cells and the fluorescence intensity of GFP positive cells were determined by FACS analysis using a CyAn ADP flow cytometer (BECKMAN COULTER). Immunoglobulin concentrations in cell culture supernatants were measured by sandwich ELISA. Slc5a6, GFP, IgG1Lc and IgG1Hc transcript accumulation was confirmed by RT-quantitative PCR assays before analyses. Surface staining, IgG titer and limiting dilution where performed according to Le Fourn et al. (2014).

### Quantitative PCR analysis

For quantitative PCR (qPCR) analysis, total RNA was extracted from 10⁶ cells and reverse transcribed into cDNA. Transcripts accumulation was quantified by qPCR using the SYBR Green-Taq polymerase kit from Eurogentec Inc and ABI Prism 7700 PCR machine (Applied Biosystems) and using primers Slc5a6-qRT-F and Slc5a6-qRT-R listed in Table 4. Transcript levels were normalized to that of GAPDH housekeeping gene.

### Statistical analysis

The results are expressed as means ± standard error of the mean (SEM). Statistical analysis was performed using the two-tailed Student's t-test. Asterisks in the figure panels refer to statistical probabilities. Statistical probability values of less than 0.05 were considered significant.

### Literature

Birch, J. R. & Racher, A. J. (2006) Antibody production. Adv Drug Deliv Rev, 58, 671-85.
Cacciatore, J. J., Chasin, L. A. & Leonard, E. F. (2010) Gene amplification and vector engineering to achieve rapid and high-level therapeutic protein production using the Dhfr-based CHO cell selection system. Biotechnol Adv, 28, 673-81.
Brunetti D, Dusi S, Morbin M, Uggetti A, Moda F, D'Amato I, Giordano C, d'Amati G, Cozzi A, Levi S, Hayflick S, Tiranti V. (2012). Pantothenate kinase-associated neurodegeneration: altered mitochondria membrane potential and defective respiration in Pank2 knock-out mouse model. Hum Mol Genet. 21:5294-305
Chusainow, J., Yang, Y. S., Yeo, J. H., Toh, P. C., et al., (2009) A study of monoclonal antibody-producing CHO cell lines: what makes a stable high producer? Biotechnol. Bioeng. 102, 1182-1196.
Dean, J. and P. Reddy (2013). "Metabolic analysis of antibody producing CHO cells in fed-batch production." Biotechnology and bioengineering 110(6): 1735-1747.
Du Z, Treiber D, McCarter JD, Fomina-Yadlin D, Saleem RA, McCoy RE, Zhang Y, Tharmalingam T, Leith M, Follstad BD, Dell B, Grisim B, Zupke C, Heath C, Morris AE, Reddy P. (2015). Use of a small molecule cell cycle inhibitor to control cell growth and improve specific productivity and product quality of recombinant proteins in CHO cell cultures. Biotechnol. Bioeng. 112: 141-155.
Garcia M., Leonardi R, Zhang YM, Rehg JE, Jackowski S. (2012). Germline deletion of pantothenate kinases 1 and 2 reveals the key roles for CoA in postnatal metabolism. PLoS One. 7:e40871.
P.A. Girod, D.Q. Nguyen, D. Calabrese, S. Puttini, M. Grandjean, D. Martinet, A. Regamey, D. Saugy, J.S. Beckmann, P. Bucher, N. Mermod (2007). Genome-wide prediction of matrix attachment regions that increase gene expression in mammalian cells. Nat. Methods, 4 (2007), pp. 747-753
Ghosal, A., N. Lambrecht, et al. (2013). "Conditional knockout of the Slc5a6 gene in mouse intestine impairs biotin absorption." American journal of physiology. Gastrointestinal and liver physiology 304(1): G64-71.
M. Grandjean, P.A. Girod, D. Calabrese, K. Kostyrko, M. Wicht, F. Yerly, C. Mazza, J.S. Beckmann, D. Martinet, N. Mermod (2011). High-level transgene expression by homologous recombination-mediated gene transfer. Nucleic Acids Res., 39, p. e104.
C.M. Hart, U.K. Laemmli (1998). Facilitation of chromatin dynamics by SARs. Curr. Opin. Genet. Dev., 8, pp. 519-525.
Jostock, T. (2011) Expression of Antibody in Mammalian Cells Cell Engineering, 7, 1-24.
V. Le Fourn, P.A. Girod, M. Buceta, A. Regamey, N. Mermod (2014). CHO cell engineering to prevent polypeptide aggregation and improve therapeutic protein secretion. Metab. Eng., 21, pp. 91-102.
Nam JH, Zhang F, Ermonval M, Linhardt RJ, Sharfstein ST. (2008). The effects of culture conditions on the glycosylation of secreted human placental alkaline phosphatase produced in Chinese hamster ovary cells. Biotechnol Bioeng 100:1178-1192.
Rothem, L., B. Berman, et al. (2005). "The reduced folate carrier gene is a novel selectable marker for recombinant protein overexpression." Molecular pharmacology 68(3): 616-624.
Sajan E, Matanguihan R, Heidemann R, Abu-Absi S, Asuncion W, Yamasaki G, Wu X, Chen J,Murphy JE, Zhang C. (2010). The effect of bioreactor pH and temperature on protein glycosylation in perfusion cultures of mammalian cells. ESACT Proc 4:785-788.
Sampathkumar SG, Jones MB,Meledeo MA, Campbell CT, Choi SS, Hida K, Gomutputra P, Sheh A, Gilmartin T, Head SR., et al. (2006). Targeting glycosylation pathways and the cell cycle: Sugar-dependent activity of butyrate-carbohydrate cancer prodrugs. Chem Biol 13:1265-1275.
Schlatter, S., Stansfield, S. H., Dinnis, D. M., Racher, A. J., et al., (2005) On the optimal ratio of heavy to light chain genes for efficient recombinant antibody production by CHO cells. Biotechnol. Prog. 21, 122-133.
Trummer E, Fauland K, Seidinger S, Schriebl K, Lattenmayer C, Kunert R, Vorauer-Uhl K, Weik R, Borth N, Katinger H, et al. (2006). Process parameter shifting: Part II. Biphasic cultivation-A tool for enhancing the volumetric productivity of batch processes using Epo-Fc expressing CHO cells. Biotechnol Bioeng 94:1045-1052.

## Claims

1. A eukaryotic expression system comprising:
- at least one first polynucleotide encoding at least one vitamin metabolic protein under the control of at least one first regulatory sequence, and
- under the control of at least one second regulatory sequence, at least one restriction enzyme cleavage site and/or at least one second polynucleotide encoding at least one product of interest,
- wherein the at least one vitamin metabolic protein is a vitamin transport protein, and wherein said vitamin transport protein is THTR-1, THTR-2, TPK, TPC and/or SMVT (sodium dependent multi vitamin transporter).

2. The eukaryotic expression system of claim 1, wherein the at least one second polynucleotide is inserted into said at least one restriction enzyme cleavage site.

3. The eukaryotic expression system of claim 1 or 2, wherein said vitamin transport protein transports a soluble vitamin such as vitamin B1, B5 and/or H.

4. The eukaryotic expression system of any one of claims 1 to 3, wherein said vitamin transport protein is SMVT.

5. The eukaryotic expression system of any one of the preceding claims, wherein the expression system is at least one expression vector.

6. The eukaryotic expression system of claim 5, wherein a singular vector comprises said at least one first and said at least one second polynucleotide.

7. The eukaryotic expression system of any one of the preceding claims, wherein said first and/or second regulatory sequence are promoters, enhancers, locus control regions (LCRs), matrix attachment regions (MARs), scaffold attachment regions (SARs), insulator elements and/or nuclear matrix-associating DNAs.

8. A kit comprising in one container, said eukaryotic expression system of any one of the preceding claims and, in a second container, instructions of how to use said system.

9. The kit of claim 8 further comprising a cell culture medium, preferably having a limiting and/or saturating concentration of at least one vitamin.

10. The kit of claim 9, wherein the cell culture medium has a limiting and/or saturating concentration of vitamin B1, B5 and/ or H.

11. A recombinant eukaryotic cell comprising the expression system of any one of claims 1 to 7; and/or having an up or down mutation of a vitamin metabolic protein, and comprising a second polynucleotide encoding a product of interest, wherein the vitamin metabolic protein is optionally intrinsic to the cell, wherein the at least one vitamin metabolic protein is a vitamin transport protein, and wherein said vitamin transport protein is THTR-1, THTR-2, TPK, TPC and/or SMVT (sodium dependent multi vitamin transporter).

12. The recombinant eukaryotic cell of claim 11, wherein the cell is a Chinese Hamster Ovary (CHO) cell.

13. The recombinant eukaryotic cell of claim 11 or 12, wherein the at least one first polynucleotide or a sequence regulating the expression the at least one first polynucleotide has an up or down mutation.

14. The recombinant eukaryotic cell of claim 11 or 12, wherein the vitamin metabolic protein interferes with vitamin metabolism and/or binds a vitamin within a cell.

15. The recombinant eukaryotic cell of claim 14, wherein the vitamin metabolic protein is pantothenate 1, 2 and/or 3 and/or is a thiamin pyrophosphate kinase, such as TPK1 (thiamin pyrophosphate kinase 1).

16. The recombinant eukaryotic cell of any one of claims 11 to 15 comprising said expression system, wherein the vitamin metabolic protein is a selectable marker for said recombinant eukaryotic cell and said recombinant eukaryotic cell produces and, preferably secretes said product of interest.

17. A eukaryotic cell culture medium comprising the recombinant eukaryotic cells according to any one of claims 11 to 16 expressing (i) a vitamin transport protein as a selectable marker and (ii) said protein of interest, wherein the at least one vitamin metabolic protein is a vitamin transport protein, and wherein said vitamin transport protein is THTR-1, THTR-2, TPK, TPC and/or SMVT (sodium dependent multi vitamin transporter), and wherein said medium is a limiting medium for B5, or a saturated medium for B5 but a limiting medium or not limiting medium for H or a saturated medium for H but a limiting medium or not limiting medium for B5, preferably a limiting medium for B5, or a saturated medium for B5 but a limiting medium for H.

18. A method for culturing and, optionally selecting recombinant eukaryotic cells comprising:
providing the expression system of any one of claims 1 to 7, preferably claims 5 and 6,
providing eukaryotic cells, wherein viability, growth and/or division of said eukaryotic cells is dependent on vitamin uptake,
introducing said expression system into said eukaryotic cells to produce said recombinant eukaryotic cells expressing said vitamin metabolic protein and said protein of interest, culturing said eukaryotic cells in a cell culture medium, and
optionally, selecting via said vitamin metabolic protein, which is preferably expressed on the surface of said recombinant eukaryotic cells, said recombinant eukaryotic cells that stably express said product of interest, wherein the at least one vitamin metabolic protein is a vitamin transport protein, and wherein said vitamin transport protein is THTR-1, THTR-2, TPK, TPC and/or SMVT (sodium dependent multi vitamin transporter).

19. The method of claim 18, wherein said medium is a limiting medium for B5, or a saturated medium for B5 but a limiting medium for H.

20. A method of producing a protein of interest, comprising:
a) transforming eukaryotic cells with an expression system of any one of claims 1 to 7 to produce recombinant eukaryotic cells;
b) culturing said recombinant eukaryotic cells in a culture medium in which viability, growth and/or division of the recombinant eukaryotic cells is dependent upon activity of one or more vitamin metabolic protein,
c) selecting for recombinant eukaryotic cells expressing said one or more vitamin metabolic protein, wherein said vitamin metabolic protein is a selectable marker to obtain selected recombinant eukaryotic cells; and
d) purifying the protein of interest from said selected recombinant eukaryotic cells or from a culture medium thereof comprising said selected recombinant eukaryotic cells.

21. The method of claim 20, wherein the vitamin metabolic protein is a vitamin transport protein preferably transporting vitamins B5, B1 and/or H and said culture medium is limiting and/or saturating for one of more of said vitamins.

22. The method of claim 21, wherein the vitamin transport protein is SMVT and said culture medium is a limiting medium for B5, or a saturated medium for B5 but a limiting medium for H.

## Patentansprüche

1. Eukaryotisches Expressionssystem, umfassend:
- mindestens ein erstes Polynucleotid, welches mindestens ein Vitaminstoffwechsel-Protein unter Steuerung durch mindestens eine erste regulatorische Sequenz codiert, und
- unter Steuerung durch mindestens eine zweite regulatorische Sequenz, mindestens eine Restriktionsenzym-Spaltstelle und/oder mindestens ein zweites Polynucleotid, das für mindestens ein Produkt von Interesse codiert,
- wobei das mindestens eine Vitaminstoffwechsel-Protein ein Vitamintransport-Protein ist und wobei das Vitamintransport-Protein THTR-1, THTR-2, TPK, TPC und/oder SMVT (natriumabhängiger Multivitamintransporter) ist.

2. Eukaryotisches Expressionssystem nach Anspruch 1, wobei das mindestens eine zweite Polynucleotid in die mindestens eine Restriktionsenzym-Spaltstelle eingeführt ist.

3. Eukaryotisches Expressionssystem nach Anspruch 1 oder 2, wobei das Vitamintransport-Protein ein lösliches Vitamin wie Vitamin B1, B5 und/oder H transportiert.

4. Eukaryotisches Expressionssystem nach einem der Ansprüche 1 bis 3, wobei das Vitamintransport-Protein SMVT ist.

5. Eukaryotisches Expressionssystem nach einem der vorhergehenden Ansprüche, wobei das Expressionssystem mindestens ein Expressionsvektor ist.

6. Eukaryotisches Expressionssystem nach Anspruch 5, wobei ein singulärer Vektor das mindestens eine erste und das mindestens eine zweite Polynucleotid umfasst.

7. Eukaryotisches Expressionssystem nach einem der vorhergehenden Ansprüche, wobei die erste und/oder zweite regulatorische Sequenz Promotoren, Enhancer, Locuskontrollregionen (LCR), Matrixanheftungsregionen (MAR), Gerüstanheftungsregionen (SAR), Isolatorelemente und/oder Kernmatrixassoziierende DNAs sind.

8. Kit, das in einem Behälter das eukaryotische Expressionssystem nach einem der vorhergehenden Ansprüche und in einem zweiten Behälter Anweisungen zur Verwendung des Systems umfasst.

9. Kit nach Anspruch 8, das weiter ein Zellkulturmedium umfasst, das vorzugsweise eine begrenzende und/oder sättigende Konzentration von mindestens einem Vitamin aufweist.

10. Kit nach Anspruch 9, wobei das Zellkulturmedium eine begrenzende und/oder sättigende Konzentration an Vitamin B1, B5 und/oder H aufweist.

11. Rekombinante eukaryotische Zelle, die das Expressionssystem nach einem der Ansprüche 1 bis 7 umfasst und/oder eine aufsteigende oder absteigende Mutation eines Vitaminstoffwechsel-Proteins aufweist, und ein zweites Polynucleotid umfasst, welches ein Produkt von Interesse codiert, wobei das Vitaminstoffwechsel-Protein optional zell-intrinsisch ist, wobei das mindestens eine Vitaminstoffwechsel-Protein ein Vitamintransport-Protein ist, und wobei das Vitamintransport-Protein THTR-1, THTR-2, TPK, TPC und/oder SMVT (natriumabhängiger Multivitamintransporter) ist.

12. Rekombinante eukaryotische Zelle nach Anspruch 11, wobei die Zelle eine Chinesischer-Hamster-Ovar (CHO)-Zelle ist.

13. Rekombinante eukaryotische Zelle nach Anspruch 11 oder 12, wobei das mindestens eine erste Polynucleotid oder eine Sequenz, welche die Expression des mindestens einen ersten Polynucleotids reguliert, eine aufsteigende oder absteigende Mutation aufweist.

14. Rekombinante eukaryotische Zelle nach Anspruch 11 oder 12, wobei das Vitaminstoffwechsel-Protein in den Vitaminstoffwechsel eingreift und/oder ein Vitamin innerhalb einer Zelle bindet.

15. Rekombinante eukaryotische Zelle nach Anspruch 14, wobei das Vitaminstoffwechsel-Protein Pantothenat 1, 2 und/oder 3 und/oder eine Thiaminpyrophosphatkinase ist, wie TPK1 (Thiaminpyrophosphatkinase 1).

16. Rekombinante eukaryotische Zelle nach einem der Ansprüche 11 bis 15, welche das Expressionssystem umfasst, wobei das Vitaminstoffwechsel-Protein ein selektierbarer Marker für die rekombinante eukaryotische Zelle ist und die rekombinante eukaryotische Zelle das Produkt von Interesse herstellt und vorzugsweise sezerniert.

17. Eukaryotisches Zellkulturmedium, das die rekombinanten eukaryotischen Zellen nach einem der Ansprüche 11 bis 16 umfasst, die (i) ein Vitamintransport-Protein als einen selektierbaren Marker und (ii) das Protein von Interesse exprimieren, wobei das mindestens eine Vitaminstoffwechsel-Protein ein Vitamintransport-Protein ist und wobei das Vitamintransport-Protein THTR-1, THTR-2, TPK, TPC und/oder SMVT (natriumabhängiger Multivitamintransporter) ist, und wobei das Medium ein begrenzendes Medium für B5, oder ein gesättigtes Medium für B5 aber ein begrenzendes Medium oder kein begrenzendes Medium für H, oder ein gesättigtes Medium für H aber ein begrenzendes Medium oder kein begrenzendes Medium für B5 ist, vorzugsweise ein begrenzendes Medium für B5, oder ein gesättigtes Medium für B5 aber ein begrenzendes Medium für H.

18. Verfahren zum Kultivieren und optional Selektieren rekombinanter eukaryotischer Zellen, umfassend:
Bereitstellen des Expressionssystems nach irgendeinem der Ansprüche 1 bis 7, vorzugsweise nach Anspruch 5 und 6,
Bereitstellen eukaryotischer Zellen, wobei die Lebensfähigkeit, das Wachstum und/oder die Teilung der eukaryotischen Zellen von der Vitaminaufnahme abhängen,
Einführen des Expressionssystems in die eukaryotischen Zellen, um die rekombinanten eukaryotischen Zellen herzustellen, die das Vitaminstoffwechsel-Protein und das Protein von Interesse exprimieren,
Kultivieren der eukaryotischen Zellen in einem Zellkulturmedium, und
optional Selektieren mittels des Vitaminstoffwechsel-Proteins, das vorzugsweise auf der Oberfläche der rekombinanten eukaryotischen Zellen exprimiert wird, der rekombinanten eukaryotischen Zellen, die das Produkt von Interesse stabil exprimieren, wobei das mindestens eine Vitaminstoffwechsel-Protein ein Vitamintransport-Protein ist und wobei das Vitamintransport-Protein THTR-1, THTR-2, TPK, TPC und/oder SMVT (natriumabhängiger Multivitamintransporter) ist.

19. Verfahren nach Anspruch 18, wobei das Medium ein begrenzendes Medium für B5 ist, oder ein gesättigtes Medium für B5 aber ein begrenzendes Medium für H ist.

20. Verfahren zur Herstellung eines Proteins von Interesse, umfassend:
a) Transformieren eukaryotischer Zellen mit einem Expressionssystem nach einem der Ansprüche 1 bis 7, um rekombinante eukaryotische Zellen herzustellen;
b) Kultivieren der rekombinanten eukaryotischen Zellen in einem Kulturmedium, in dem die Lebensfähigkeit, das Wachstum und/oder die Teilung der rekombinanten eukaryotischen Zellen von der Aktivität eines oder mehrerer Vitaminstoffwechsel-Proteine abhängen,
c) Selektieren rekombinanter eukaryotischer Zellen, die das eine oder die mehreren Vitaminstoffwechsel-Proteine exprimieren, wobei das Vitaminstoffwechsel-Protein ein selektierbarer Marker ist, um selektierte rekombinante eukaryotische Zellen zu erhalten; und
d) Reinigen des Proteins von Interesse von den selektierten rekombinanten eukaryotischen Zellen oder von einem Kulturmedium davon, das die selektierten rekombinanten eukaryotischen Zellen umfasst.

21. Verfahren nach Anspruch 20, wobei das Vitaminstoffwechsel-Protein ein Vitamintransport-Protein ist, das vorzugsweise Vitamin B5, B1 und/oder H transportiert, und das Kulturmedium für eines oder mehrere der genannten Vitamine begrenzend und/oder sättigend ist.

22. Verfahren nach Anspruch 21, wobei das Vitamintransport-Protein SMVT ist, und das Kulturmedium ein begrenzendes Medium für B5 ist, oder ein gesättigtes Medium für B5 aber ein begrenzendes Medium für H ist.

## Revendications

1. Système d'expression eucaryote comprenant :
- au moins, un premier polynucléotide codant, au moins, une protéine métabolique de vitamine sous le contrôle, d'au moins, une première séquence régulatrice, et
- sous le contrôle, d'au moins, une seconde séquence régulatrice, d'au moins, un site de clivage enzymatique de restriction et/ou, d'au moins, un second polynucléotide codant, au moins, un produit d'intérêt,
- dans lequel ladite protéine métabolique de vitamine est une protéine de transport de vitamines, et ladite protéine de transport de vitamines est THTR-1, THTR-2, TPK, TPC et/ou SMVT (transporteur pour plusieurs vitamines dépendant du sodium).

2. Système d'expression eucaryote, selon la revendication 1, dans lequel au moins ledit second polynucléotide est inséré dans au moins ledit site de clivage enzymatique de restriction.

3. Système d'expression eucaryote, selon la revendication 1 ou 2, dans lequel ladite protéine de transport de vitamines transporte une vitamine soluble telle que la vitamine B1, B5 et/ou H.

4. Système d'expression eucaryote, selon l'une quelconque des revendications 1 à 3, dans lequel ladite protéine de transport de vitamines est la SMVT.

5. Système d'expression eucaryote, selon l'une quelconque des revendications précédentes, dans lequel le système d'expression est, au moins, un vecteur d'expression.

6. Système d'expression eucaryote, selon la revendication 5, dans lequel un vecteur singulier comprend, au moins, ledit premier polynucléotide et, au moins, ledit second polynucléotide.

7. Système d'expression eucaryote, selon l'une quelconque des revendications précédentes, dans lequel lesdites première et/ou seconde séquences régulatrices sont des promoteurs, des amplificateurs, des régions de contrôle de locus (LCR), des régions de fixation de matrice (MAR), des régions de fixation à l'armature (SAR), des éléments isolateurs et/ou des ADN d'association à une matrice nucléaire.

8. Kit comprenant, dans un récipient, ledit système d'expression eucaryote, selon l'une quelconque des revendications précédentes et, dans un second récipient, des instructions sur le mode d'emploi dudit système.

9. Kit selon la revendication 8, comprenant en outre un milieu de culture cellulaire, ayant de préférence une concentration limitante et/ou saturante, d'au moins, une vitamine.

10. Kit selon la revendication 9, dans lequel le milieu de culture cellulaire a une concentration limitante et/ou saturante de vitamine B1, B5 et/ou H.

11. Cellule eucaryote recombinante comprenant le système d'expression, selon l'une quelconque des revendications 1 à 7 ; et/ou ayant une mutation ascendante ou descendante d'une protéine métabolique de vitamine, et comprenant un second polynucléotide codant un produit d'intérêt, dans laquelle la protéine métabolique de vitamine est facultativement intrinsèque à la cellule, dans laquelle, au moins, une protéine métabolique de vitamine est une protéine de transport de vitamine, et dans laquelle ladite protéine de transport de vitamine est THTR-1, THTR-2, TPK, TPC et/ou SMVT (transporteur pour plusieurs vitamines dépendant du sodium).

12. Cellule eucaryote recombinante, selon la revendication 11, dans laquelle ladite cellule est une cellule d'ovaire de hamster chinois (CHO).

13. Cellule eucaryote recombinante, selon la revendication 11 ou 12, dans laquelle, au moins, un premier polynucléotide ou une séquence régulant l'expression d'au moins un premier polynucléotide a une mutation ascendante ou descendante.

14. Cellule eucaryote recombinante, selon la revendication 11 ou 12, dans laquelle la protéine métabolique de vitamine interfère avec le métabolisme des vitamines et/ou lie une vitamine à l'intérieur d'une cellule.

15. Cellule eucaryote recombinante, selon la revendication 14, dans laquelle la protéine métabolique de vitamine est du pantothénate 1, 2 et/ou 3 et/ou est une thiamine pyrophosphate kinase, telle que TPK1 (thiamine pyrophosphate kinase 1).

16. Cellule eucaryote recombinante, selon l'une quelconque des revendications 11 à 15, comprenant ledit système d'expression, dans laquelle la protéine métabolique de vitamine est un marqueur sélectionnable pour ladite cellule eucaryote recombinante et ladite cellule eucaryote recombinante produit et, de préférence, sécrète ledit produit d'intérêt.

17. Milieu de culture de cellules eucaryotes comprenant les cellules eucaryotes recombinantes, selon l'une quelconque des revendications 11 à 16, exprimant (i) une protéine de transport de vitamines en tant que marqueur sélectionnable et (ii) ladite protéine d'intérêt, dans lequel au moins une protéine métabolique de vitamine est une protéine de transport de vitamines, et dans lequel ladite protéine de transport de vitamines est THTR-1, THTR-2, TPK, TPC et/ ou SMVT (transporteur pour plusieurs vitamines dépendant du sodium), et dans lequel ledit milieu est un milieu limitant pour B5, ou un milieu saturé pour B5 mais un milieu limitant ou non limitant pour H, ou un milieu saturé pour H mais un milieu limitant ou non limitant pour B5, de préférence, un milieu limitant pour B5, ou un milieu saturé pour B5 mais un milieu limitant pour H.

18. Procédé pour cultiver et, facultativement, sélectionner des cellules eucaryotes recombinantes comprenant :
la fourniture du système d'expression, selon l'une quelconque des revendications 1 à 7, de préférence, des revendications 5 et 6,
la fourniture de cellules eucaryotes, dans lequel la viabilité, la croissance et/ou la division desdites cellules eucaryotes dépend de l'absorption de vitamines,
l'introduction dudit système d'expression dans lesdites cellules eucaryotes pour produire lesdites cellules eucaryotes recombinantes exprimant ladite protéine métabolique de vitamine et ladite protéine d'intérêt,
la culture desdites cellules eucaryotes dans un milieu de culture cellulaire, et
facultativement, la sélection, par l'intermédiaire de ladite protéine métabolique de vitamine, qui est de préférence exprimée à la surface desdites cellules eucaryotes recombinantes, lesdites cellules eucaryotes recombinantes qui expriment, de manière stable, ledit produit d'intérêt, dans lequel ladite protéine métabolique de vitamine est une protéine de transport de vitamines, et dans lequel ladite protéine de transport de vitamines est THTR-1, THTR-2, TPK, TPC et/ou SMVT (transporteur pour plusieurs vitamines dépendant du sodium).

19. Procédé, selon la revendication 18, dans lequel ledit milieu est un milieu limitant pour B5, ou un milieu saturé pour B5 mais un milieu limitant pour H.

20. Procédé de production d'une protéine d'intérêt, comprenant :
a) la transformation des cellules eucaryotes avec un système d'expression, selon l'une quelconque des revendications 1 à 7, pour produire des cellules eucaryotes recombinantes ;
b) la culture desdites cellules eucaryotes recombinantes, dans un milieu de culture, dans lequel la viabilité, la croissance et/ ou la division des cellules eucaryotes recombinantes dépend de l'activité d'une ou de plusieurs protéines métaboliques de vitamine,
c) la sélection des cellules eucaryotes recombinantes exprimant ladite ou lesdites protéines métaboliques de vitamine, où ladite protéine métabolique de vitamine est un marqueur sélectionnable pour obtenir des cellules eucaryotes recombinantes sélectionnées ; et
d) la purification de la protéine d'intérêt desdites cellules eucaryotes recombinantes sélectionnées ou d'un milieu de culture de celles-ci comprenant lesdites cellules eucaryotes recombinantes sélectionnées.

21. Procédé, selon la revendication 20, dans lequel la protéine métabolique de vitamine est une protéine de transport de vitamines transportant de préférence les vitamines B5, B1 et/ ou H, et ledit milieu de culture est limitant et/ ou saturant pour une ou plusieurs desdites vitamines.

22. Procédé, selon la revendication 21, dans lequel la protéine de transport de vitamines est SMVT et ledit milieu de culture est un milieu limitant pour B5, ou un milieu saturé pour B5 mais un milieu limitant pour H.
